# EUROPEAN PATENT APPLICATION

(11) **EP 4 635 421 A1**
(43) Date of publication of application: **22.10.2025**
(21) Application number: 24189490.6
(22) Date of filing: 18.07.2024
(51) Int. Cl.: A61B 8/00, A61B 5/00

(54) **ULTRASOUND IMAGING APPARATUS FOR PROVIDING BODY MARKER AND CONTROL METHOD THEREOF**

(30) Priority: 17.04.2024 KR 20240051337
(71) Applicant: Samsung Medison Co., Ltd., Hongcheon-gun, Gangwon-do 25108 (KR)
(72) Inventor: GONG, Soyeon, 05340 Seoul (KR); KIM, Mose, 05340 Seoul (KR)
(74) Representative: Frey, Sven Holger

(57) **Abstract**

An ultrasound imaging apparatus may include a probe configured to transmit an ultrasound signal to an object and detect an echo signal reflected from the object, a display, a communication module, a memory storing at least one instruction, and at least one processor. The at least one processor may be configured to execute the at least one instruction to generate a first ultrasound image from ultrasound data generated by the probe, receive sensing data generated by detecting at least one part of the object and the probe from a sensing apparatus through the communication module, generate, based on the sensing data, a body marker representing a scanning part of the object and a probe marker positioned on the body marker to represent a position of the probe on the scanning part of the object, display the first ultrasound image, the body marker, and the probe marker on the display, and change the body marker based on at least one of a shape, a type, or a scanning direction of the scanning part or a patient's position.

## Description

### BACKGROUND

### 1. Field

An embodiment relates to an ultrasound imaging apparatus capable of providing a body marker, a method of controlling the ultrasound imaging apparatus, and a computer-readable recording medium storing a program for performing the method of controlling the ultrasound imaging apparatus on a computer.

### 2. Description of the Related Art

Recently, in the medical field, various medical imaging apparatuses have been widely used to image and obtain information about biological tissues of the human body for the purpose of early diagnosis of various diseases or surgery. Representative examples of such medical imaging apparatuses include ultrasound imaging apparatuses, computed tomography (CT) apparatuses, and magnetic resonance imaging (MRI) apparatuses.

An ultrasound imaging apparatus irradiates an ultrasound signal generated from a transducer of a probe to an object and receives information about the signal reflected from the object to non-invasively obtain at least one image about an internal area (for example, a soft tissue or blood flow) of the object. Ultrasound imaging apparatuses can be used for various medical purposes, such as observing the interior of an object, detecting foreign materials, and measuring injuries. Advantages of the ultrasound imaging apparatuses include being more reliable than imaging apparatuses using X rays, displaying images in real time, and being safe as there is no patient radiation exposure. Therefore, the ultrasound imaging apparatuses are widely used together with other imaging apparatuses.

Because an ultrasound imaging apparatus is used to scan local areas of a, the scanning areas depend on the positions of a probe. Due to this characteristic, a technology that shows the position of the probe when an ultrasound image is captured has been proposed. However, because ultrasound images captured by the probe depend on various factors including the position of the probe and the patient's position, there is a demand for a technology that correctly provides information about the position of the probe when an ultrasound image is captured.

### SUMMARY

Additional aspects will be set forth in part in the description which follows and, in part, will be apparent from the description, or may be learned by practice of the presented embodiments of the disclosure.

According to an aspect of an embodiment, an ultrasound imaging apparatus may be provided. The ultrasound imaging apparatus may include a probe configured to transmit an ultrasound signal to an object and detect an echo signal reflected from the object, a display, a communication module, a memory storing at least one instruction, and at least one processor. The at least one processor may be configured to execute the at least one instruction to generate a first ultrasound image from ultrasound data generated by the probe, receive sensing data generated by detecting at least a part of the object and the probe from a sensing apparatus through the communication module, generate, based on the sensing data, a body marker and a probe marker, the body marker representing a scanning part of the object and the probe marker being positioned on the body marker to represent a position of the probe on the scanning part of the object, display the first ultrasound image, the body marker, and the probe marker on the display, and change the body marker based on at least one of a shape, a type, or a scanning direction of the scanning part or a patient's position.

Also, according to an embodiment, the at least one processor may be further configured to execute the at least one instruction to update the body marker and the probe marker based on the sensing data, synchronize the body marker, the probe marker, and the ultrasound image with each other, and display the body marker, the probe marker, and the ultrasound image.

Also, according to an embodiment, the at least one processor may be further configured to execute the at least one instruction to select, based on the sensing data, a candidate body marker from among a plurality of candidate body markers, as a body marker, and locate the probe marker on the selected body marker, wherein the plurality of candidate body markers may be expressed differently according to at least one among a shape, a type, or a scanning direction of the scanning part, or a patient's positions.

Also, according to an embodiment, the body marker and the probe marker may be generated by the sensing apparatus, and the at least one processor may be further configured to execute the at least one instruction to receive the body marker and the probe marker generated by the sensing apparatus through the communication module, and display the received body marker and the received probe marker on the display.

Also, according to an embodiment, the at least one processor is further configured to execute the at least one instruction to synchronize an operation time of the sensing apparatus with an operation time of the ultrasound imaging apparatus, synchronize an acquisition time of the sensing data with an acquisition time of the first ultrasound image, and display the body marker and the probe marker generated from the synchronized sensing data, together with the first ultrasound image.

Also, according to an embodiment, the at least one processor may be further configured to execute the at least one instruction to synchronize the body marker, the probe marker, and the first ultrasound image with each other by using time information included in the sensing data and time information included in the first ultrasound image.

Also, according to an embodiment, the sensing apparatus may include a glass device including a detecting sensor, and the detecting sensor may include at least one of an image sensor, an infrared sensor, a thermal imaging camera, an optical camera, a stereo camera, or an ultrasonic sensor.

Also, according to an embodiment, the glass device may be configured to transmit the sensing data to the ultrasound imaging apparatus while a user's gaze is directed toward the scanning part, and stop transmitting the sensing data to the ultrasound imaging apparatus while the user's gaze is directed toward another part except for the scanning part. Also, the at least one processor may be further configured to execute the at least one instruction to update the body marker and the probe marker based on the sensing data while receiving the sensing data, and stop updating the body marker and the probe marker while reception of the sensing data stops.

Also, according to an embodiment, the at least one processor may be further configured to execute the at least one instruction to input the sensing data to a first artificial intelligence model to obtain the body marker, wherein the sensing data may be input to the first artificial intelligence model in a form of a one-dimensional array.

Also, according to an embodiment, a position and an orientation of the probe marker on the body marker may be defined based on an anatomical feature of the body marker.

Also, according to an embodiment, the at least one processor may be further configured to execute the at least one instruction to stop displaying the probe marker in response to determining, based on the sensing data, that the probe deviates from the scanning part.

Also, according to an embodiment, the at least one processor may be further configured to execute the at least one instruction to generate a Graphic User Interface (GUI) view that displays the body marker and the ultrasound image in such a way as to overlap the body marker with the ultrasound image, and display the GUI view on the display.

Also, according to an embodiment, the at least one processor may be further configured to execute the at least one instruction to display, on the body marker, a scan area indicator indicating an area scanned by the probe.

Also, according to an embodiment, the scanning part may be the breast.

Also, according to an embodiment, the body marker may include a three-dimensional body marker.

Also, according to an embodiment, the at least one processor may be further configured to execute the at least one instruction to transmit the first ultrasound image to the sensing apparatus, through the communication module, and the sensing apparatus may be configured to display the body marker and the first ultrasound image as Augmented Reality (AR) objects.

Also, according to an embodiment, the ultrasound imaging apparatus may further include a sensing apparatus.

Also, according to an embodiment, a method of controlling an ultrasound imaging apparatus may be provided. The method of controlling an ultrasound imaging apparatus may include generating a first ultrasound image from ultrasound data generated by a probe, receiving, from a sensing apparatus, sensing data generated by detecting at least a part of an object and the probe, generating, based on the sensing data, a body marker and a probe marker, the body marker representing a scanning part of the object and the probe marker positioned on the body marker to represent a position of the probe on the scanning part of the object, displaying the first ultrasound image, the body marker, and the probe marker, and changing the body marker based on at least one of a shape, a type, or a scanning direction of the scanning part or a patient's position.

According to another aspect of an embodiment, a computer-readable recording medium storing a program for performing a method of controlling an ultrasound imaging apparatus on a computer may be provided.

### BRIEF DESCRIPTION OF THE DRAWINGS

The above and other aspects, features, and advantages of certain embodiments of the disclosure will be more apparent from the following description taken in conjunction with the accompanying drawings, in which:
The disclosure may be easily understood by combining the following detailed description with the accompanying drawings, and reference numerals represent structural elements.
FIG. 1A is block diagrams showing a configuration of an ultrasound imaging system according to an embodiment;
FIG. 1B is block diagrams showing a configuration of an ultrasound imaging system according to an embodiment;
FIG. 2A shows an ultrasound imaging system according to an embodiment;
FIG. 2B shows an ultrasound imaging system according to an embodiment;
FIG. 2C shows an ultrasound imaging system according to an embodiment;
FIG. 2D shows an ultrasound imaging system according to an embodiment;
FIG. 3 shows an operation of an ultrasound imaging apparatus according to an embodiment;
FIG. 4 is a block diagram showing a structure of an ultrasound imaging apparatus according to an embodiment;
FIG. 5 is a flowchart illustrating a method of controlling an ultrasound imaging apparatus, according to an embodiment;
FIG. 6 shows a process of generating a body marker from sensing data, according to an embodiment;
FIG. 7 shows a process of selecting a body marker from among a plurality of candidate body markers, according to an embodiment;
FIG. 8 shows a process of generating a body marker by using an artificial intelligence (Al) model, according to an embodiment;
FIG. 9 shows a process of generating an ultrasound image, a body marker, and a probe marker, synchronized with each other, according to an embodiment;
FIG. 10 is a flowchart illustrating a method of synchronizing a body marker, a probe marker, and an ultrasound image with each other, according to an embodiment;
FIG. 11 is a flowchart illustrating a method of displaying an ultrasound image, a body marker, and a probe marker, according to an embodiment;
FIG. 12 shows a Graphic User Interface (GUI) for displaying a body marker and an ultrasound image, according to an embodiment;
FIG. 13 shows a configuration of displaying a medical image of another modality, a body marker, and a probe marker, according to an embodiment;
FIG. 14 shows a GUI for representing scan information on a body marker, according to an embodiment;
FIG. 15 is a flowchart illustrating a process of displaying a body marker and an ultrasound image in a sensing apparatus, according to an embodiment;
FIG. 16A shows body markers and probe markers according to an embodiment;
FIG. 16B shows body markers and probe markers according to an embodiment;
FIG. 16C shows body markers and probe markers according to an embodiment; and
FIG. 17 shows a process of obtaining sensing data and obtaining a body marker and a probe marker, according to an embodiment.

### DETAILED DESCRIPTION

The present disclosure clarifies the scope of rights of the claims of the disclosure and explains the principles of the embodiments to enable those skilled in the technical art to which the embodiments of the disclosure pertain to practice the embodiments of the disclosure. The embodiments of the disclosure may be implemented in various forms.

Throughout this specification, like reference numerals will refer to like components. The present specification does not describe all elements of the embodiments, and descriptions about general content in the technical art to which the disclosure pertains or overlapping content between the embodiments will be omitted. As used herein, the terms "module" or "unit" may be implemented as one or a combination of two or more of software, hardware, or firmware, and according to some embodiments, a plurality of "modules" or "units" may be implemented as a single element, or a single "module" or "unit" may include a plurality of elements.

The singular form of a noun corresponding to an item may include one or a plurality of the items unless clearly indicated otherwise in a related context.

In the disclosure, wordings such as "A or B", "at least one of A and B", "at least one of A or B," "A, B or C," "at least one of A, B and C," and "at least one of A, B, or C" may include any one or all possible combinations of items listed together in the corresponding phrase among the phrases.

The term "and/or" includes any or all combinations of a plurality of associated listed components.

Terms such as "first", "second", or "1^{st}" or "2^{nd}" may be used simply to distinguish a component from other components, without limiting the component in other aspects (e.g., importance or order).

Also, the terms 'front surface', 'rear surface', 'upper surface', 'lower surface', 'side surface', 'left side', 'right side', 'upper portion', 'lower portion', etc., as used in the disclosure, are defined based on the drawings, and the shapes and positions of the components are not limited by the terms.

It will be understood that when the terms "includes," "comprises," "including," and/or "comprising," when used in the disclosure, specify the presence of stated features, figures, steps, operations, components, members, or combinations thereof, but do not preclude the presence or addition of one or more other features, figures, steps, operations, components, members, or combinations thereof.

It will be understood that when a certain component is referred to as being "connected to", "coupled to", "supported by" or "in contact with" another component, it can be directly or indirectly connected to, coupled to, supported by, or in contact with the other component. When a component is indirectly connected to, coupled to, supported by, or in contact with another component, it may be connected to, coupled to, supported by, or in contact with the other component through a third component.

It will also be understood that when a certain component is referred to as being "on" or "over" another component, it can be directly on the other component or intervening components may also be present.

Hereinafter, an ultrasound imaging apparatus according to various embodiments will be described in detail with reference to the accompanying drawings. In the following description with reference to the accompanying drawings, the same or corresponding components are assigned similar reference numerals, and overlapping descriptions thereof may be omitted.

In the disclosure, an image may include a medical image obtained by a medical imaging apparatus, such as a magnetic resonance imaging (MRI) apparatus, a computed tomography (CT) apparatus, an ultrasound imaging apparatus, an X-ray imaging apparatus, and the like.

In the disclosure, 'object' may be a target to be photographed, and include a human, an animal, or a part thereof. For example, an object may include a part (intestines, organs, etc.) of a body, a phantom, etc.

In the disclosure, an "ultrasound image" means an image about an object generated or processed based on an ultrasound signal transmitted to the object and then reflected from the object.

An embodiment may be provided to more accurately store and provide a photographing condition when an ultrasound image is captured by providing a body marker to which a patient's position or features have been reflected when the ultrasound image is captured.

Hereinafter, embodiments will be described in detail.

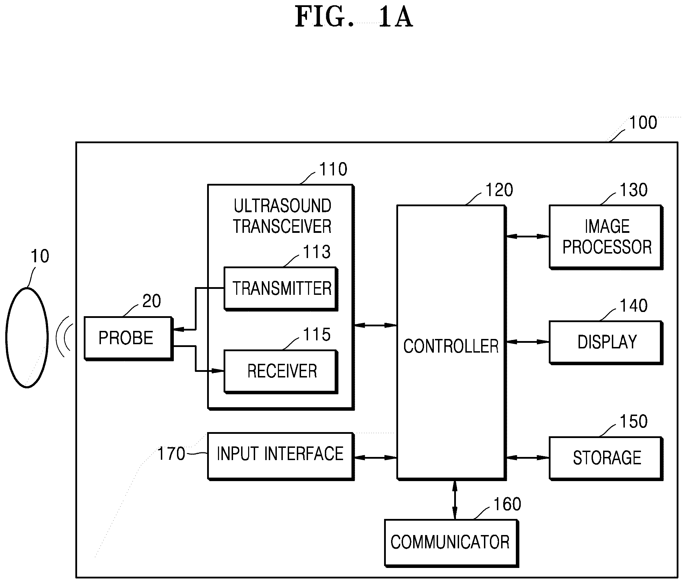

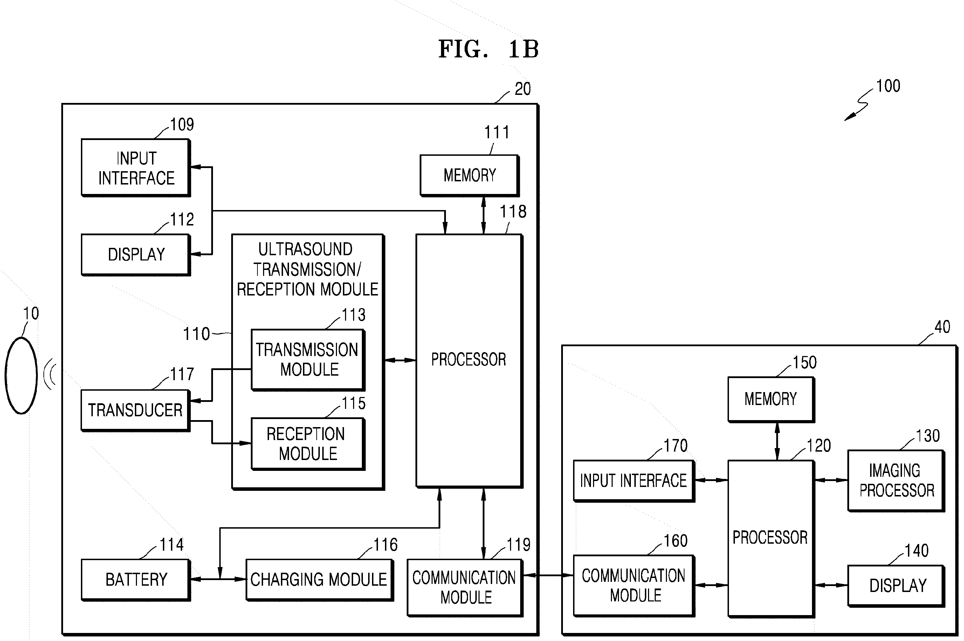

FIGS. 1A and 1B are block diagrams showing configurations of an ultrasound imaging system according to an embodiment.

Referring to FIGS. 1A and 1B, an ultrasound imaging system 100 may include a probe 20 and an ultrasound imaging apparatus 40.

The ultrasound imaging apparatus 40 may be implemented as a cart type or a portable type. Examples of portable ultrasound imaging apparatuses may include a smart phone, a laptop computer, a personal digital assistant (PDA), a tablet PC, etc., each of which includes a probe and an application, although not limited thereto. The ultrasound imaging apparatus 40 may be implemented as a probe-integrated type.

The probe 20 may include a wired probe connected to the ultrasound imaging apparatus 40 by wire to communicate with the ultrasound imaging apparatus 40 by wire, a wireless probe wirelessly connected to the ultrasound imaging apparatus 40 to wirelessly communicate with the ultrasound imaging apparatus 40, and/or a hybrid probe connected to the ultrasound imaging apparatus 40 by wire or wirelessly to communicate with the ultrasound imaging apparatus 40 by wire or wirelessly.

According to various embodiments, as shown in FIG. 1A, the ultrasound imaging apparatus 40 may include an ultrasound transmission/reception module 110, or the probe 20 may include the ultrasound transmission/reception module 110 as shown in FIG. 1B. According to various embodiments, both the ultrasound imaging apparatus 40 and the probe 20 may include the ultrasound transmission/reception module 110.

According to various embodiments, the probe 20 may include at least one of an imaging processor 130, a display 140, an input interface 170, or a combination thereof. In the disclosure, descriptions about the ultrasound transmission/reception module 110, the imaging processor 130, the display 140, or the input interface 170 included in the ultrasound imaging apparatus 40 may be respectively applied to the ultrasound transmission/reception module 110, the imaging processor 130, the display 140, or the input interface 170 included in the probe 20.

FIG. 1A is a block diagram showing a configuration of the ultrasound imaging system 100 when the probe 20 is a wired probe or a hybrid probe.

The probe 20 may include a plurality of transducers. The plurality of transducers may be arranged in a preset arrangement and implemented as a transducer array. The transducer array may correspond to a one-dimensional (1D) array or a two-dimensional (2D) array. The plurality of transducers may transmit an ultrasound signal to an object 10 according to a transmission signal received from a transmission module 113. The plurality of transducers may form a reception signal in response to receiving an ultrasound signal (an echo signal) reflected from the object 10. Also, the probe 20 may be implemented as an integrated type integrated into the ultrasound imaging apparatus 40 or a separated type connected to the ultrasound imaging apparatus 40 by wire. Also, the ultrasound imaging apparatus 40 may be connected to a single or plurality of probes 20 according to an implementation type.

In the case in which the probe 20 is a wired probe or a hybrid probe, the probe 20 may include a cable and a connector that are connectable to a connector of the ultrasound imaging apparatus 40.

The probe 20 according to an embodiment may be implemented as a two-dimensional probe. In the case in which the probe 20 is a two-dimensional probe, the plurality of transducers included in the probe 20 may form a two-dimensional transducer array by being arranged two-dimensionally.

For example, the two-dimensional transducer array may be configured by arranging a plurality of sub arrays each including a plurality of transducers arranged in a first direction in a second direction that is different from the first direction.

Also, in the case in which the probe 20 according to an embodiment is a two-dimensional probe, the ultrasound transmission/reception module 110 may include at least one of an analog beamformer or a digital beamformer. Also, according to an embodiment, the two-dimensional probe may include at least one of an analog beamformer or a digital beamformer, or a combination thereof according to an implementation type.

The processor 120 may control the transmission module 113 to form a transmission signal that is to be applied to each of a plurality of transducers 117 included in the probe 20 in consideration of positions and focus points of the transducers 117.

The processor 120 may control the reception module 115 to perform analog-to-digital conversion on reception signals received from the probe 20, sum the digital-converted reception signals in consideration of the positions and focus points of the plurality of transducers 117, and thereby generate ultrasound data.

In the case in which the probe 20 is implemented as a two-dimensional probe, the processor 120 may calculate a time delay value for digital beamforming for each of a plurality of sub arrays included in a two-dimensional transducer array. Also, the processor 120 may calculate a time delay value for analog beamforming for each of transducers included in any sub array among the plurality of sub arrays. The processor 120 may control the analog beamformer and the digital beamformer to form a transmission signal that is to be transmitted to each of the plurality of transducers according to time delay values for analog beamforming and time delay values for digital beamforming. Also, the processor 120 may control the analog beamformer to sum signals received from the plurality of transducers for each sub array according to the time delay values for analog beamforming. Also, the processor 120 may control the ultrasound transmission/reception module 110 to perform analog-to-digital conversion on signals summed for the respective sub arrays. Also, the processor 120 may control the digital beamformer to sum the digital-converted signals according to the time delay values for digital beamforming and generate ultrasound data.

The imaging processor 130 may generate or process an ultrasound image by using the generated ultrasound data.

The display 140 may display the generated ultrasound image and various information processed in the ultrasound imaging apparatus 40 or the probe 20. The probe 20 or the ultrasound imaging apparatus 40 may include a single or plurality of displays 140 according to an implementation type. Also, the display 140 may include a touch panel or a touch screen. Also, the display 140 may include a flexible display.

The processor 120 may control overall operations of the ultrasound imaging apparatus 40, and control operations of components of the ultrasound imaging apparatus 40. The processor 120 may execute a program or instructions stored in the memory 150 to perform or control various operations or functions of the ultrasound imaging apparatus 40. Also, the processor 120 may receive a control signal from the input interface 170 or an external device to control an operation of the ultrasound imaging apparatus 40.

The ultrasound imaging apparatus 40 may include a communication module 160, and be connected to and communicate with an external device (for example, the probe 20, a server, a medical device, or a portable device (a smart phone, a tablet PC, a wearable device, etc.)) through the communication module 160.

The communication module 160 may include one or more components for enabling communication with an external device. The communication module 160 may include at least one of, for example, a short-range communication module, a wired communication module, or a wireless communication module.

The communication module 160 may receive a control signal or data from an external device. The processor 120 may control an operation of the ultrasound imaging apparatus 40 according to a control signal received through the communication module 160. Also, the processor 120 may transmit a control signal to the external device through the communication module 160 and control the external device according to the transmitted control signal. The external device may operate according to the control signal received from the ultrasound imaging apparatus 40, or process data received from the ultrasound imaging apparatus 40.

A program or application related to the ultrasound imaging apparatus 40 may be installed in the external device. The program or application installed in the external device may control the ultrasound imaging apparatus 40, or operate according to a control signal or data received from the ultrasound imaging apparatus 40.

The external device may receive or download the program or application related to the ultrasound imaging apparatus 40 from the ultrasound imaging apparatus 40, the probe 20, or a server, install the program or application therein, and execute the program or application. The ultrasound imaging apparatus 40, the probe 20, or the server, which provides the program or application, may include a recording medium that stores an instruction, a command, an installation file, an execution file, or related data of the corresponding program or application. The external device may be sold with the program or application installed.

The memory 150 may store various data or programs for driving and controlling the ultrasound imaging apparatus 40, input/output ultrasound data, and ultrasound images.

The input interface 170 may receive a user input for controlling the ultrasound imaging apparatus 40. For example, the user input may include an input of operating a button, a key pad, a mouse, a trackball, a jog switch, a knop, etc., an input of touching a touch pad or a touch screen, a voice input, a motion input, a biometric information input (for example, iris recognition, fingerprint recognition, etc.), etc., although not limited thereto.

FIG. 1B is a control block diagram of the ultrasound imaging system 100 with the probe 20 which is a wireless probe or a hybrid probe.

According to various embodiments, the ultrasound imaging apparatus 40 shown in FIG. 1B may be replaced with the ultrasound imaging apparatus 40 described with reference to FIG. 1A.

According to various embodiments, the probe 20 shown in FIG. 1A may be replaced with the probe 20 which will be described with reference to FIG. 1B.

The probe 20 may include a display 112, a transmission module 113, a battery 114, a transducer 117, a charging module 116, a reception module 115, an input interface 109, a producer 118, and a communication module 119. FIG. 1B shows a case in which the probe 20 includes both the transmission module 113 and the reception module 115. However, the probe 20 may include a part of components of the transmission module 113 and the reception module 117, and another part of the components of the transmission module 113 and the reception module 117 may be included in the ultrasound imaging apparatus 40. Also, according to an embodiment, the probe 20 may further include the imaging processor 130.

The transducer 117 may include a plurality of transducers. The plurality of transducers may be arranged in a preset arrangement and implemented as a transducer array. The transducer array may correspond to a 1D array or a 2D array. The plurality of transducers may transmit an ultrasound signal to an object 10 according to a transmission signal applied from the transmission module 113. Also, the plurality of transducers may receive an ultrasound signal reflected from the object 10 and form or generate an electrical reception signal.

The charging module 116 may charge the battery 114. The charging module 116 may receive power from outside. According to an embodiment, the charging module 116 may receive power wirelessly. Also, according to an embodiment, the charging module 116 may receive power by wire. The charging module 116 may transfer the received power to the battery 114.

The processor 118 may control the transmission module 113 to generate or form a transmission signal that is to be applied to each of the plurality of transducers in consideration of positions and focus points of the plurality of transducers.

The processor 118 may control the reception module 115 to perform analog-to-digital conversion on reception signals received from the transducer 117, sum the digital-converted reception signals in consideration of the positions and focus points of the plurality of transducers, and thereby generate ultrasound data. According to an embodiment, the probe 20 may include the imaging processor 130, and in this case, the probe 20 may generate an ultrasound image by using the generated ultrasound data.

In the case in which the probe 20 is implemented as a 2D probe, the processor 118 may calculate a time delay value for digital beamforming for each of a plurality of sub arrays included in a 2D transducer array. Also, the processor 118 may calculate a time delay value for analog beamforming for each of transducers included in any sub array among the plurality of sub arrays. The processor 118 may control an analog beamformer and a digital beamformer to form a transmission signal that is to be applied to each of the plurality of transducers, according to time delay values for analog beamforming and time delay values for digital beamforming. Also, the processor 118 may control the analog beamformer to sum signals received from the plurality of transducers for each sub array according to the time delay values for analog beamforming. Also, the processor 118 may control the ultrasound transmission/reception module 110 to perform analog-to-digital conversion on signals summed for the respective sub arrays. Also, the processor 118 may control the digital beamformer to sum the digital-converted signals according to the time delay value for digital beamforming and generate ultrasound data.

The processor 118 may control overall operations of the probe 20 and control operations of components of the probe 20. The processor 118 may execute a program or instructions stored in the memory 111 to perform or control various operations or functions of the probe 20. Also, the processor 118 may control an operation of the probe 20 by receiving a control signal from the input interface 109 of the probe 20 or an external device (for example, the ultrasound imaging apparatus 40). Also, the processor 118 may control an operation of the probe 20 by receiving a control signal from the input interface 108 or an external device. The input interface 109 may receive a user input for controlling the probe 20. For example, the user input may include an input of operating a button, a key pad, a mouse, a trackball, a jog switch, a knop, etc., an input of touching a touch pad or a touch screen, a voice input, a motion input, a biometric information input (for example, iris recognition, fingerprint recognition, etc.), etc., although not limited thereto.

The display 112 may display an ultrasound image generated by the probe 20, an ultrasound image generated by processing ultrasound data generated by the probe 20, an ultrasound image received from the ultrasound imaging apparatus 40, or various information processed in the ultrasound imaging system 100. Also, the display 112 may further display state information of the probe 20. The state information of the probe 20 may include at least one of device information of the probe 20, battery state information of the probe 20, frequency band information of the probe 20, output information of the probe 20, abnormality information of the probe 20, setting information of the probe 20, or temperature information of the probe 20.

The probe 20 may include a single or plurality of displays 112 according to an implementation type. Also, the display 112 may include a touch panel or a touch screen. Also, the display 112 may include a flexible display.

The communication module 119 may wirelessly transmit the generated ultrasound data or ultrasound image to the ultrasound imaging apparatus 40 through a wireless network. Also, the communication module 119 may receive a control signal and data from the ultrasound imaging apparatus 40.

The ultrasound imaging apparatus 40 may receive ultrasound data or an ultrasound image from the probe 20.

In an embodiment, according to the probe 20 including the imaging processor 130 capable of generating an ultrasound image by using ultrasound data, the probe 20 may transmit ultrasound data or an ultrasound image generated by the imaging processor 130 to the ultrasound imaging apparatus 40.

In an embodiment, according to the probe 20 not including the imaging processor 130 capable of generating an ultrasound image by using ultrasound data, the probe 20 may transmit ultrasound data to the ultrasound imaging apparatus 40. The ultrasound data may include ultrasound raw data, and the ultrasound image may be ultrasound image data.

The ultrasound imaging apparatus 40 may include the processor 120, the imaging processor 130, the display 140, the memory 150, the communication module 160, and the input interface 170.

The imaging processor 130 may generate or process an ultrasound image by using ultrasound data received from the probe 20.

The display 140 may display an ultrasound image received from the probe 20, an ultrasound image generated by processing ultrasound data received from the probe 20, or various information processed in the ultrasound imaging system 100. The ultrasound imaging apparatus 40 may include a single or plurality of displays 140 according to an implementation type. Also, the display 140 may include a touch panel or a touch screen. Also, the display 140 may include a flexible display.

The processor 120 may control overall operations of the ultrasound imaging apparatus 40, and control operations of components of the ultrasound imaging apparatus 40. The processor 120 may execute a program or instruction stored in the memory 150 to perform or control various operations or functions of the ultrasound imaging apparatus 40. Also, the processor 120 may control an operation of the ultrasound imaging apparatus 40 by receiving a control signal from the input interface 170 or an external device.

The ultrasound imaging apparatus 40 may include the communication module 160, and be connected to and communicate with an external device (for example, the probe 20, a server, a medical device, a portable device (a smart phone, a tablet PC, a wearable device, etc.) through the communication module 160.

The communication module 160 may include one or more components for enabling communication with an external device. The communication module 160 may include at least one of, for example, a short-range communication module, a wired communication module, or a wireless communication module.

The communication module 160 of the ultrasound imaging apparatus 40 may communicate with the communication module 119 of the probe 20 through a network or a short-range wireless communication method. For example, the communication module 160 of the ultrasound imaging apparatus 40 may communicate with the communication module 119 of the probe 20 through any one of wireless data communication methods including wireless local area network (LAN), wireless-fidelity (Wi-Fi), Bluetooth, zigbee, Wi-Fi Direct (WFD), infrared communication (infrared Data Association (IrDA)), Bluetooth LowEnergy (BLE), Near Field Communication (NFC), Wireless Broadband Internet (Wibro), World Interoperability for Microwave Access (WiMAX), Shared Wireless Access Protocol (SWAP), Wireless Gigabit Allicance (WiGig), radio frequency (RF) communication, or 60GHz mm Wave short-distance communication.

To this end, the communication module 160 of the ultrasound imaging apparatus 40 and the communication module 119 of the probe 20 may include at least one of a wireless LAN communication module, a Wi-Fi communication module, a Bluetooth communication module, a zigbee communication module, a WFD communication module, an IrDA communication module, a BLE communication module, a NFC communication module, a Wibro communication module, a WiMAX communication module, a SWAP communication module, a WiGig communication module, an RF communication module, or a 60GHz mm Wave short-distance communication module.

In an embodiment, the probe 20 may transmit device information (for example, identification (ID) information) of the probe 20 to the ultrasound imaging apparatus 40 by using a first communication method (for example, BLE) and be wirelessly paired with the ultrasound imaging apparatus 40. Also, the probe 20 may transmit ultrasound data and/or an ultrasound image to the paired ultrasound imaging apparatus 40.

The device information of the probe 20 may include various information related to a serial number, model number, battery state, etc. of the probe 20.

The ultrasound imaging apparatus 40 may receive the device information (for example, ID information) of the probe 20 from the probe 20 by using the first communication method (for example, BLE), and be wirelessly paired with the probe 20. Also, the ultrasound imaging apparatus 40 may transmit an activation signal to the paired probe 20 and receive ultrasound data and/or an ultrasound image from the probe 20. At this time, the activation signal may include a signal for controlling an operation of the probe 20.

In an embodiment, the probe 20 may transmit the device information (for example, ID information) of the probe 20 to the ultrasound imaging apparatus 40 by using the first communication method (for example, BLE), and be wirelessly paired with the ultrasound imaging apparatus 40. Also, the probe 20 may transmit ultrasound data and/or an ultrasound image to the ultrasound imaging apparatus 40 paired by the first communication method by using a second communication method (for example, 60 GHz mm wave or Wi-Fi).

The ultrasound imaging apparatus 40 may receive the device information (for example, ID information) of the probe 20 from the probe 20 by using the first communication method (for example BLE), and be wirelessly paired with the probe 20. Also, the ultrasound imaging apparatus 40 may transmit an activation signal to the paired probe 20, and receive ultrasound data and/or an ultrasound image from the probe 20 by using the second communication method (for example, 60 GHz mm Wave or Wi-Fi).

According to an embodiment, the first communication method used for pairing of the probe 20 and the ultrasound imaging apparatus 40 may have a lower frequency band than that of the second communication method by which the probe 20 transmits ultrasound data and/or an ultrasound image to the ultrasound imaging apparatus 40.

The display 140 of the ultrasound imaging apparatus 40 may display user interfaces (Uls) representing the device information of the probe 20. For example, the display 140 may display ID information of the probe 20 as a wireless ultrasound probe, a pairing method used for pairing with the probe 20, a data communication state between the probe 20 and the ultrasound imaging apparatus 40, a method for data communication with the ultrasound imaging apparatus 40, or a UI representing a battery state of the probe 20.

According to the probe 20 including the display 112, the display 112 of the probe 20 may display a UI representing the device information of the probe 20. For example, the display 112 may display ID information of the probe 20 as a wireless ultrasound probe, a pairing method used for pairing with the probe 20, a data communication state between the probe 20 and the ultrasound imaging apparatus 40, a method for data communication with the ultrasound imaging apparatus 40, or a UI representing a battery state of the probe 20.

The communication module 160 may receive a control signal or data from an external device. The processor 120 may control an operation of the ultrasound imaging apparatus 40 according to a control signal received through the communication module 160.

Also, the processor 120 may transmit a control signal to an external device through the communication module 160 to control the external device according to the control signal. The external device may operate according to the control signal received from the ultrasound imaging apparatus 40 or process data received from the ultrasound imaging apparatus 40.

The external device may receive or download a program or application related to the ultrasound imaging apparatus 40 from the ultrasound imaging apparatus 40, the probe 20, or a server, install the program or application therein, and execute the program or application. The ultrasound imaging apparatus 40, the probe 20, or the server, which provides the program or application, may include a recording medium that stores an instruction, command, installation file, execution file, related data, etc. of the corresponding program or application. The external device may be sold with the program or application installed.

The memory 150 may store various data or programs for driving and controlling the ultrasound imaging apparatus 40, input/output ultrasound data, ultrasound images, etc.

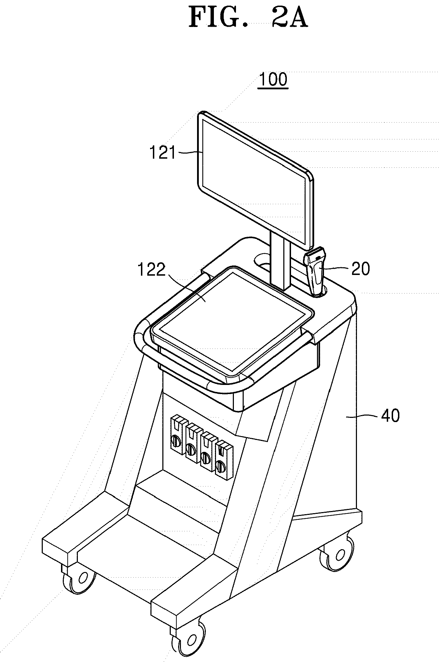

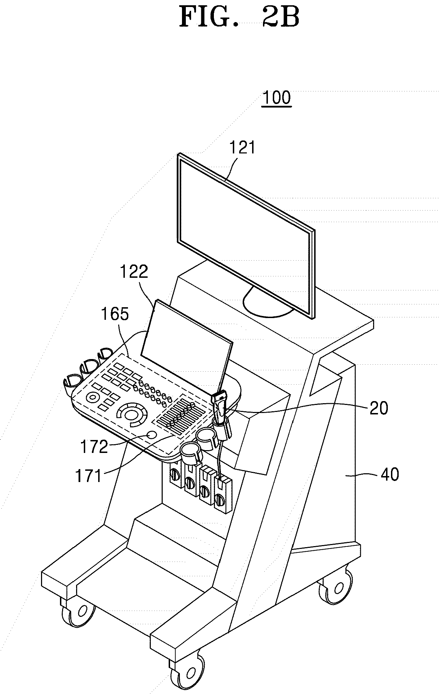

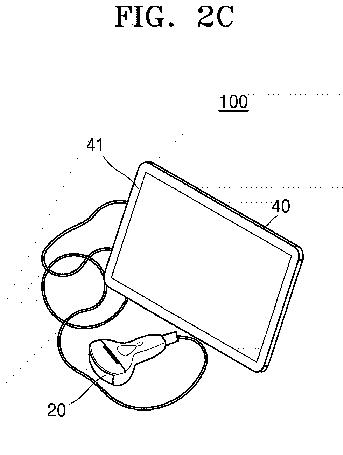

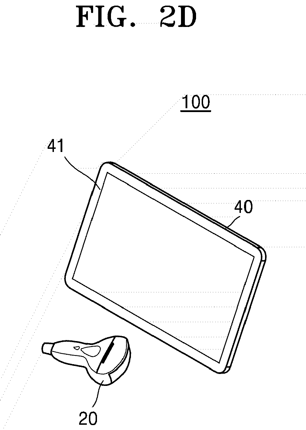

Examples of the ultrasound imaging system 100 according to an embodiment will be described with reference to FIGS. 2A, 2B, 2C, and 2D, below.

FIGS. 2A, 2B, 2C, and 2D show ultrasound imaging apparatuses according to an embodiment.

Referring to FIGS. 2A and 2B, each of ultrasound imaging apparatuses 40a and 40b may include a main display 121 and a sub display 122. The main display 121 and the sub display 122 may correspond to the display 140 of FIGS. 1A and 1B. At least one of the main display 121 or the sub display 122 may be implemented as a touch screen. At least one of the main display 121 or the sub display 122 may display ultrasound images or various information processed in the ultrasound imaging apparatuses 40a and 40b. Also, at least one of the main display 121 or the sub display 122 may be implemented as a touch screen and provide a Graphic User Interface (GUI) to receive data for controlling the ultrasound imaging apparatuses 40a and 40b from a user. For example, the main display 121 may display an ultrasound image, and the sub display 122 may display a control panel for controlling a display of the ultrasound image in the form of a GUI. The sub display 122 may receive control data for controlling a display of an ultrasound image through the control panel displayed in the form of the GUI. For example, a Time Gain Compensation (TGC) button, a Lateral Gain Compensation (LGC) button, a Freeze button, a trackball, a jog switch, or a knop may be provided as a GUI to the sub display 122.

The ultrasound imaging apparatuses 40a and 40b may control the display of the ultrasound image displayed on the main display 121 by using the received control data. Also, the ultrasound imaging apparatuses 40a and 40b may be connected to the probe 20 by wire or wirelessly to transmit/receive an ultrasound signal to/from an object.

Referring to FIG. 2B, the ultrasound imaging apparatus 40b may further include a control panel 165, in addition to the main display 121 and the sub display 122. The control panel 165 may include a button, a trackball, a jog switch, a knop, etc., and receive data for controlling the ultrasound imaging apparatus 40b from a user. For example, the control panel 165 may include a TGC button 171, a Freeze button 172, etc. The TGC button 171 may be a button for setting a TGC value for each depth of an ultrasound image. Also, according to a detection of an input to the Freeze button 171 while the ultrasound imaging apparatus 40b scans an ultrasound image, the ultrasound imaging apparatus 40b may maintain a display state of a frame image at the corresponding time, capture the frame image at the corresponding time, or store the frame image at the corresponding time.

Meanwhile, the button, trackball, jog switch, knop, etc. included in the control panel 165 may be provided as a GUI to the main display 121 or the sub display 122. Also, the ultrasound imaging apparatuses 40a and 40b may be connected to the probe 20 to transmit/receive an ultrasound signal to/from an object.

Also, the ultrasound imaging apparatuses 40a and 40b may include an input/output interface which may be one(s) of various types, such as a speaker, a Light Emitting Diode (LED), a vibrating device, etc. For example, the ultrasound imaging apparatuses 40a and 40b may output various information in the form of graphics, sound, or vibration through the input/output interface. Also, the ultrasound imaging apparatuses 40a and 40b may output various notifications or data through the input/output interface.

Referring to FIGS. 2C and 2D, ultrasound imaging apparatuses 40c and 40d may be implemented as portable types. Examples of the ultrasound imaging apparatuses 40c and 40d which are portable types may include a smart phone, a laptop computer, a PDA, or a tablet PC, which includes a probe and an application, although not limited thereto.

The ultrasound imaging apparatus 40c may include a main body 41. Referring to FIG. 2C, the probe 20 may be connected to one side of the main body 41 by wire. To this end, the main body 41 may include a connection terminal to/from which a cable connected to the probe 20 is attachable/detachable. The probe 20 may include a cable including a connection terminal that is connectable to the main body 41.

Referring to FIG. 2D, the probe 20 may be connected to the ultrasound imaging apparatus 40 wirelessly. The main body 41 may include an input/output interface (for example, a touch screen). The input/output interface may display an ultrasound image, various information processed in the ultrasound image, a GUI, etc.

The ultrasound imaging apparatus 40 may establish communication with the probe 20 or be paired with the probe 20 by using short-range wireless communication. For example, the ultrasound imaging apparatus 40 may communicate with the probe 20 by using Bluetooth, BLE, Wi-Fi, or WFD.

The ultrasound imaging apparatus 40 may execute a program or application related to the probe 20 to control the probe 20 and output information related to the probe 20. The ultrasound imaging apparatus 40 may perform an operation related to the probe 20 by communicating with a preset server. The probe 20 may be registered in the ultrasound imaging apparatuses 40c and 40d or the preset server. The ultrasound imaging apparatus 40 may communicate with the probe 20 registered therein and perform an operation related to the probe 20.

Also, the ultrasound imaging apparatus 40 may include an input/output interface which may be one(s) of various types, such as a speaker, a LED, a vibrating device, etc. For example, the ultrasound imaging apparatus 40 may output various information in the form of graphics, sound or vibration through the input/output interface. Also, the ultrasound imaging apparatus 40 may output various notifications or data through the input/output interface.

According to an embodiment, the ultrasound imaging apparatus 40 may process an ultrasound image or obtain additional information from an ultrasound image by using an Artificial Intelligence (Al) model. According to an embodiment, the ultrasound imaging apparatus 40 may generate an ultrasound image or perform processing, such as compensation, image quality enhancement, encoding, or decoding, on an ultrasound image, by using an Al model. Also, according to an embodiment, the ultrasound imaging apparatus 40 may perform processing, such as defining a base line, obtaining anatomical information, obtaining lesion information, extracting a plane, defining a boundary, measuring a length, measuring a width, measuring a volume, or generating an annotation, on an ultrasound image, by using the Al model.

The Al model may be included in the ultrasound imaging apparatus 40 or the server.

The Al model may be implemented by using various Artificial Neural Network (ANN) models or Deep Neural Network (DNN) models. Also, the Al model may be learned and generated by using various machine learning algorithms or deep learning algorithms. The Al model may be implemented by using a model, such as, for example, a Convolutional Neural Network (CNN), a Recurrent Neural Network (RNN), a Generative Adversarial Network (GAN), a Long Short-Term Memory (LSTM), etc.

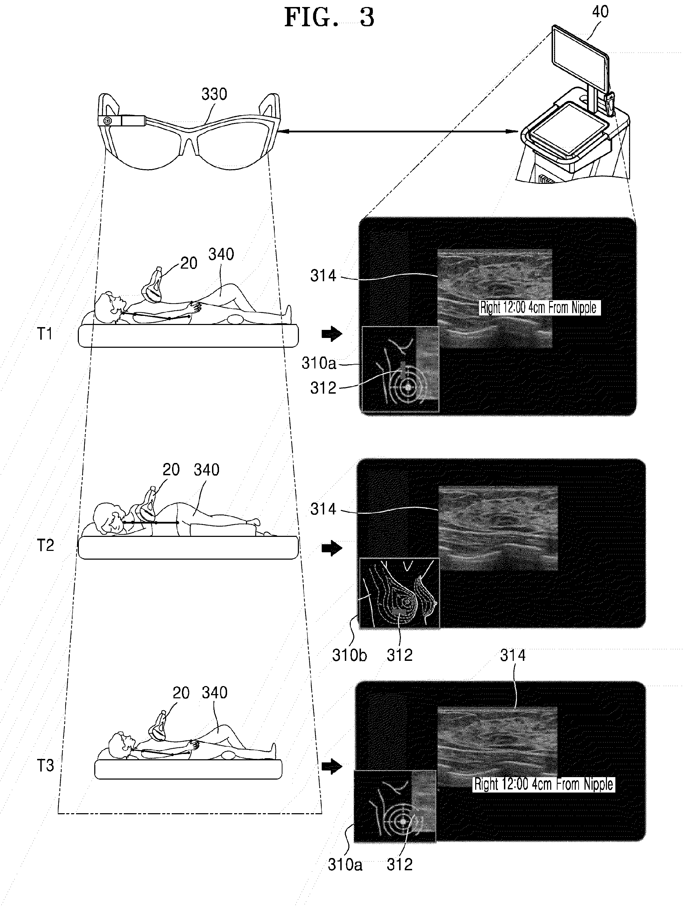

FIG. 3 shows an operation of an ultrasound imaging apparatus according to an embodiment.

According to an embodiment, the ultrasound imaging apparatus 40 may perform short-range communication with a sensing apparatus 330. The sensing apparatus 330 may generate sensing data by detecting a patient 340 and the probe 20. The sensing apparatus 330 may generate sensing data by detecting a scene where a medical personnel scans the patient 340 using the probe 20. The sensing apparatus 330 may transmit the sensing data to the ultrasound imaging apparatus 40. The sensing apparatus 330 may communicate with the ultrasound imaging apparatus 40 by wire or wirelessly. The sensing apparatus 330 may be included in the ultrasound imaging apparatus 40 or separated from the ultrasound imaging apparatus 40. The sensing apparatus 330 may be implemented as any one of various apparatuses, such as a wearable device, a communication terminal, a camera, an infrared sensor, a heat detector, or an ultrasound sensor. According to an embodiment, the sensing apparatus 330 may be implemented in the form of a glass apparatus that a medical personnel wears. The glass apparatus may include an optical camera, and in this case, the sensing data may correspond to a photographed image photographed by the optical camera.

According to an embodiment, the ultrasound imaging apparatus 40 may display body markers 310a and 310b and a probe marker 312, together with an ultrasound image 314. The ultrasound image 314 may correspond to an ultrasound image photographed in real time or an ultrasound image 314 previously photographed and stored. The body markers 310a and 310b may be drawings or images obtained by simplifying a shape of a scanning part of the patient 340. For example, according to the scanning part being the breast, the body markers 310a and 310b may be expressed as shown in FIG. 3. The probe marker 312 may be an indicator indicating a position of the probe 20 on a scanning part of an object. The probe marker 312 may indicate a position and an orientation of the probe 20 while the ultrasound image 314 is captured, on the body markers 310a and 310b. A shape of the probe marker 312 may be set to any one of various shapes, and for example, the probe marker 312 may be expressed as a shape corresponding to the shape of the probe 20, such as a quadrangle or an oval.

According to an embodiment, the scanning part may correspond to the breast, arm, or leg.

According to an embodiment, the ultrasound imaging apparatus 40 may generate the body markers 310a and 310b to which a state of the patient 340 has been reflected upon scanning of the patient 340, by using sensing data. According to an embodiment, the ultrasound imaging apparatus 40 may select and display the body markers 310a and 310b by reflecting the state of the patient 340 upon ultrasound scanning of the patient 340. The body markers 310a and 310b may be selected based on at least one of a shape, a type, or a scanning direction of the scanning part or a position of the patient 340 upon scanning of the patient 340. For example, according to ultrasound scanning on the patient 340 who lies looking at the ceiling at a first time T1, a first body marker 310a may be displayed, and according to ultrasound scanning on the patient 340 who lies on his/her side at a second time T2, a second body marker 310b may be displayed.

In an example in which a scanning part is the breast, a shape, a type, and a scanning direction of the scanning part and a patient's position will be described. The shape of the scanning part may be defined as Relaxed, Round, Side Set, Slender, Asymmetric, Athletic, Bell, East West, or Tear Drop. The type of the scanning part may be defined as normal breast, left excised breast, or right excised breast. The scanning direction may be defined as left breast or right breast. The patient's position may be defined as lying looking at the ceiling, lying on the right side, or lying on the left side.

In the disclosure, the body markers 310a and 310b may be collectively referred to as reference numeral 310.

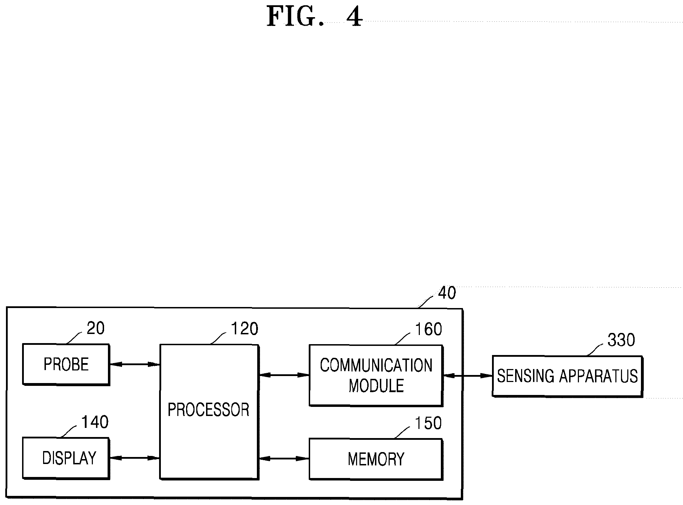

FIG. 4 is a block diagram showing a structure of an ultrasound imaging apparatus according to an embodiment.

According to an embodiment, the ultrasound imaging apparatus 40 may include the probe 20, the processor 120, the display 140, the memory 150, and the communication module 160.

The processor 120 may control overall operations of the ultrasound imaging apparatus 40, and control operations of components of the ultrasound imaging apparatus 40. The processor 120 may execute a program or instructions stored in the memory 150 to perform or control various operations or functions of the ultrasound imaging apparatus 40. Also, the processor 120 may receive a control signal from the input interface 170 or an external device to control an operation of the ultrasound imaging apparatus 40.

The memory 150 may store various data or programs for driving and controlling the ultrasound imaging device 40, input/output ultrasound data, and ultrasound images.

The display 140 may display an ultrasound image, and various information processed in the ultrasound imaging apparatus 40 or the probe 20. The probe 20 or the ultrasound imaging apparatus 40 may include a single or plurality of displays 140 according to an implementation type. Also, the display 140 may include a touch panel or a touch screen. Also, the display 140 may include a flexible display.

According to an embodiment, the communication module 160 may communicate with the sensing apparatus 330. The communication module 160 may communicate with the sensing apparatus 330 by wire or wirelessly. The communication module 160 may communicate with the sensing apparatus 330 through short-range communication. For example, the communication module 160 may communicate with the sensing apparatus 330 through any one of wireless data communication methods including wireless local area network (LAN), wireless-fidelity (Wi-Fi), Bluetooth, zigbee, Wi-Fi Direct (WFD), infrared communication (infrared Data Association (IrDA)), Bluetooth LowEnergy (BLE), Near Field Communication (NFC), Wireless Broadband Internet (Wibro), World Interoperability for Microwave Access (WiMAX), Shared Wireless Access Protocol (SWAP), Wireless Gigabit Allicance (WiGig), radio frequency (RF) communication, or 60GHz mm Wave short-distance communication.

According to an embodiment, the communication module 160 may establish a short-range communication channel by pairing with the sensing apparatus 330. For example, the communication module 160 may establish a Bluetooth communication channel with the sensing apparatus 330.

The ultrasound imaging apparatus 400 may receive sensing data from the sensing apparatus 330. The ultrasound imaging apparatus 40 may receive sensing data from the sensing apparatus 330 through a communication channel established with the sensing apparatus 330. The ultrasound imaging apparatus 40 may perform a preset security authentication process with the sensing apparatus 330, and, according to the security authentication process being successful, the ultrasound imaging device 40 may receive sensing data from the sensing apparatus 330.

The processor 120 may generate a body marker 310 and a probe marker 312 based on the sensing data. The processor 120 may select the body marker 310 based on at least one of a shape, a type, or a scanning direction of a scanning part or a patient's position. According to an embodiment, the ultrasound imaging apparatus 40 may store a plurality of candidate body markers. The processor 120 may set the body marker 310 by selecting one from the plurality of candidate body markers based on at least one of a shape, a type, or a scanning direction of a scanning part or a patient's position.

The processor 120 may recognize the probe 20 from the sensing data and recognize a position and an orientation of the probe 20. The processor 120 may recognize the probe 20 from the sensing data by using a preset object recognition algorithm. The processor 120 may recognize the position and orientation of the probe 20 based on an anatomical feature of the scanning part. For example, according to the scanning part being the breast, the processor 120 may recognize a position and an orientation of the probe 20 based on the nipples of the breast. The processor 120 may recognize an anatomical feature from the sensing data, and recognize a position and an orientation of the probe 20 based on the recognized anatomical feature. For example, the processor 120 may define the position of the probe 20 as 20 cm to the right and 3cm upward from the nipple. The processor 120 may locate the probe marker 312 on the selected body marker 310. The processor 120 may locate the probe marker 312 on the body marker 310 based on the position and orientation of the probe 20.

In an embodiment, according to a scanning part being the breast, an anatomical feature of the scanning part may include at least one of the nipples, upper arms, armpits, collarbone, head, or navel. The processor 120 may define a position of the probe 20 by using a plurality of anatomical features. For example, the processor 120 may recognize a position of the probe 20 by using the nipple, head, and navel as anatomical features.

In an embodiment, according to a scanning part being the arm, an anatomical feature of the scanning part may include the elbow. In an embodiment, according to a scanning part being the leg, an anatomical feature of the scanning part may include the knee.

The processor 120 may generate an ultrasound image from ultrasound data generated by the probe 20. The processor 120 may synchronize a time of the ultrasound image with times of the body marker 310 and the probe marker 312. The processor 120 may display the ultrasound image, the body marker 310, and the probe marker 312, synchronized with each other, together. Also, the processor 120 may store the ultrasound image, the body marker 310, and the probe marker 312, synchronized with each other, together, in the memory 150.

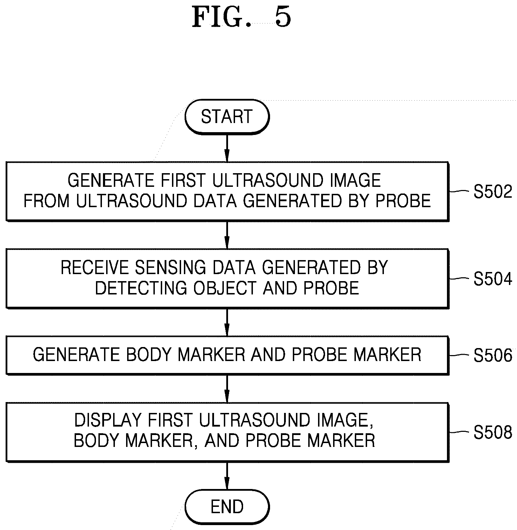

FIG. 5 is a flowchart illustrating a method of controlling an ultrasound imaging apparatus, according to an embodiment.

Operations of the method of controlling the ultrasound imaging apparatus, according to an embodiment, may be performed by various types of ultrasound imaging apparatuses. In the disclosure, an embodiment in which the ultrasound imaging apparatus 40 according to embodiments performs the method of controlling the ultrasound imaging apparatus will be described. Accordingly, embodiments described with respect to the ultrasonic imaging apparatus 40 are applicable to embodiments with respect to the method of controlling the ultrasonic imaging apparatus, and also, embodiments described with respect to the method of controlling the ultrasonic imaging apparatus are applicable to embodiments with respect to the ultrasonic imaging apparatus 40. The method of controlling the ultrasound imaging apparatus according to embodiments is not limited to being performed by the ultrasound imaging apparatus 400 according to the disclosure, and may be performed by various types of ultrasound imaging apparatuses.

In operation S502, the ultrasound imaging apparatus 40 may generate a first ultrasound image from ultrasound data generated by the probe 20. The ultrasound imaging apparatus 40 may generate a two-dimensional ultrasound image or a three-dimensional image by using the ultrasound data. Also, the ultrasound imaging apparatus 40 may generate a moving image or a still image from the ultrasound data.

Then, in operation S504, the ultrasound imaging apparatus 40 may receive sensing data generated by detecting at least one part of an object and the probe 20, from the sensing apparatus 330.

According to an embodiment, the sensing data may correspond to a camera image captured by an optical camera, an infrared image, a thermal image, or an ultrasound image. The camera image captured by the optical camera may correspond to an optical image generated from light in a visible light region.

Also, according to an embodiment, the sensing data may include a body marker 310 and a probe marker 312. The sensing apparatus 330 may generate the body marker 310 and the probe marker 312 from the sensing data. The sensing apparatus 330 may transmit the body marker 310 and the probe marker 312 to the ultrasound imaging apparatus 40.

Also, according to an embodiment, the sensing data may include time information about a time at which the sensing data has been obtained. Because the sensing data is synchronized with the ultrasound image, the time information of the sensing data may be used for synchronization with the ultrasound image. The sensing apparatus 330 may insert the time information into the sensing data and transmit the sensing data including the time information to the ultrasound imaging apparatus 40.

Then, in operation S506, the ultrasound imaging apparatus 40 may generate a body marker 310 and a probe marker 312 from the sensing data.

According to an embodiment, the sensing data may correspond to a camera image, an infrared image, a thermal image, or an ultrasound image. The ultrasound imaging apparatus 40 may recognize a scanning part of the object and the probe 20 from the sensing data.

The ultrasound imaging apparatus 40 may generate the body marker 310 according to a result of recognition of the scanning part of the object. The ultrasound imaging apparatus 40 may select one of a plurality of candidate body markers as the body marker 310. The plurality of candidate body markers may include a plurality of body markers expressed differently according to shapes, types, and directions of the scanning part of the object, and a patient's positions. For example, according to the scanning part being the breast, the candidate body markers may be divided into the left breast and the right breast. Also, the candidate body markers may be divided into lying looking at the ceiling, lying on the side, etc. Also, the candidate body markers may be divided into the case in which one breast has been removed by mastectomy, the case in which both breasts have been removed by mastectomy, the case in which a breast implant has been inserted, etc.

According to an embodiment, the ultrasound imaging apparatus 40 may select a body marker by using a plurality of anatomical features. Also, according to an embodiment, the ultrasound imaging apparatus 40 may identify at least one of a shape, a type, or a direction of the scanning part or the patient's position by using a plurality of anatomical features. For example, according to the scanning part being the breast, the plurality of anatomical features may include at least one of the nipples, upper arms, armpits, collarbone, head, or navel.

For example, in the case in which a patient lies on the bed looking at the ceiling (Supine position) and an observer stands next to the patient while looking at the patient, the upper arms may be on the left and the armpits may be relatively on the right. In this case, the near breast may be the patient's right breast and the far side may be the patient's left breast. Accordingly, the ultrasound imaging apparatus 40 may recognize the upper arms, armpits, and nipples from the sensing data, and distinguish between the left breast and the right breast based on positions of the recognized upper arms, armpits, and nipples.

Also, according to an observer seeing the head on the left and the navel on the right, the near breast may be the patient's right breast, and the far side may be the patient's left breast. The ultrasound imaging apparatus 40 may recognize the head, navel, and nipples from the sensing data, and distinguish between the left breast and the right breast based on positions of the recognized head, navel, and nipples.

For example, according to a patient lying on the bed looking at the ceiling (Supine position) and an observer standing next to the patient while looking at the patient, the head may be located to the left side of the nipples and the navel may be located to the right side of the nipples. In this case, the nipple closer to the observer may be the right ripple and another one farther from the observer may be the left ripple. Also, parts of the nipples toward the head may be upper parts of the nipples and other parts of the nipples toward the navel may be lower parts of the nipples. The ultrasound imaging apparatus 40 may recognize the head, navel, and nipples from the sensing data, and recognize upper, lower, left, and right directions with respect to the nipples based on positions of the recognized head, navel, and nipples.

Also, for example, according to a scanning part being the leg, an anatomical feature of the scanning part may include at least one of the knees, hip joints, or ankles. For example, a case in which the blood vessels of the leg are scanned using the probe 20 and an observer stands next to a lying patient while looking at the patient is assumed. In this case, the hip joints may be located to the left of the knees and the ankles may be located to the right of the knees. Then, the leg closer to the observer may be the right leg, and the leg farther from the observer may be the left leg. The ultrasound imaging apparatus 40 may recognize the knees and hip joints from the sensing data, and recognize the right leg and the left leg based on positions of the recognized knees and hip joints.

According to an embodiment, the ultrasound imaging apparatus 40 may generate a probe marker 312 according to the recognized result by the probe 20. The ultrasound imaging apparatus 40 may recognize a position and an orientation of the probe 20 from the sensing data. The ultrasound imaging apparatus 40 may locate the probe marker 312 based on the position and orientation of the probe 20 on the selected body marker 310. The ultrasound imaging apparatus 40 may locate the probe marker 312 on the body marker 310 based on an anatomical feature of a scanning part of an object. For example, according to the scanning part of the object being the breast, the ultrasound imaging apparatus 40 may define a position of the probe marker 312 and locate the probe marker 312 on the body marker 310 based on positions of the nipples.

According to an embodiment, the sensing data may include the body marker 310 and the probe marker 312 generated by the sensing apparatus 330. The ultrasound imaging apparatus 40 may use the body marker 310 and the probe marker 312 included in the sensing data. The ultrasound imaging apparatus 40 may use the body marker 310 and the probe marker 312 included in the sensing data without any processing or after post-processing.

Then, in operation S508, the ultrasound imaging apparatus 40 may display an ultrasound image 314, the body marker 310, and the probe marker 312 on the display 140. The ultrasound image 314, the body marker 310, and the probe marker 312 may be synchronized with each other and then displayed.

The ultrasound imaging apparatus 40 may update the ultrasound image 314, the body marker 310, and the probe marker 312 while performing an ultrasound scanning process. The ultrasound imaging apparatus 40 may update the ultrasound image 314, the body marker 310, and the probe marker 312 based on ultrasound data and sensing data received in real time. Also, according to reception of a freeze signal, the ultrasound imaging apparatus 40 may display the ultrasound image 314, the body marker 310, and the probe marker 312 at a time at which the freeze signal is received, at a still state.

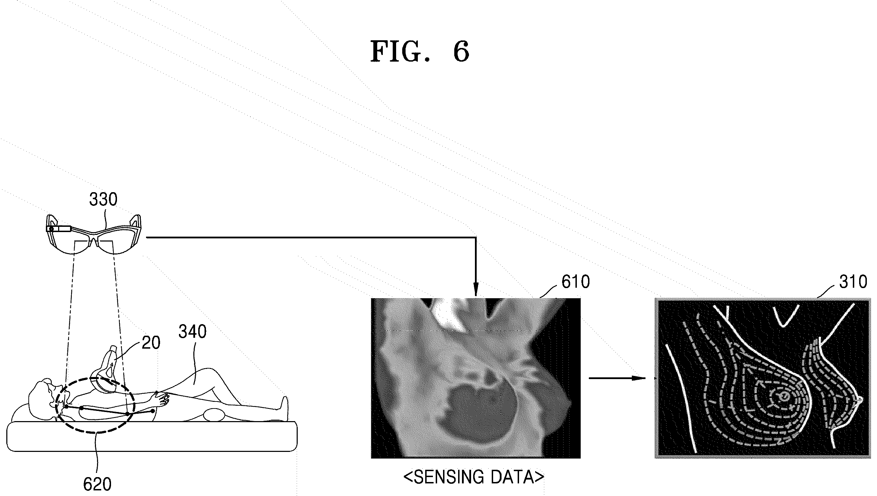

FIG. 6 shows a process of generating a body marker from sensing data, according to an embodiment.

According to an embodiment, the sensing apparatus 330 may generate sensing data 610 by photographing a scanning part 620 of a patient 340 and the probe 20. According to an embodiment, the sensing data 610 may be generated as three-dimensional data.

According to an embodiment, the sensing apparatus 330 may include a stereo camera. The sensing apparatus 330 may generate the three-dimensional sensing data 610 by using the stereo camera.

The sensing apparatus 330 or the ultrasound imaging apparatus 40 may generate a body marker 310 from the sensing data 610. The sensing apparatus 330 or the ultrasound imaging apparatus 40 may compare the sensing data 610 with a plurality of candidate body markers and select a candidate body marker having highest similarity to the sensing data 610 as the body marker 310. According to an embodiment, the body marker 310 may be expressed in two or three dimensions. According to the sensing data 330 corresponding to three-dimensional data, the body marker 310 may correspond to two or three dimensions.

Hereinafter, an embodiment in which the ultrasound imaging apparatus 40 generates a body marker 310 and a probe marker 312 from sensing data 610 will be described. However, this is for convenience of description, and the embodiment in which the ultrasound imaging apparatus 40 generates the body marker 310 and the probe marker 312 from the sensing data 610 may also be applicable to an embodiment in which the sensing apparatus 330 generates a body marker 310 and a probe marker 312 from sensing data 610.

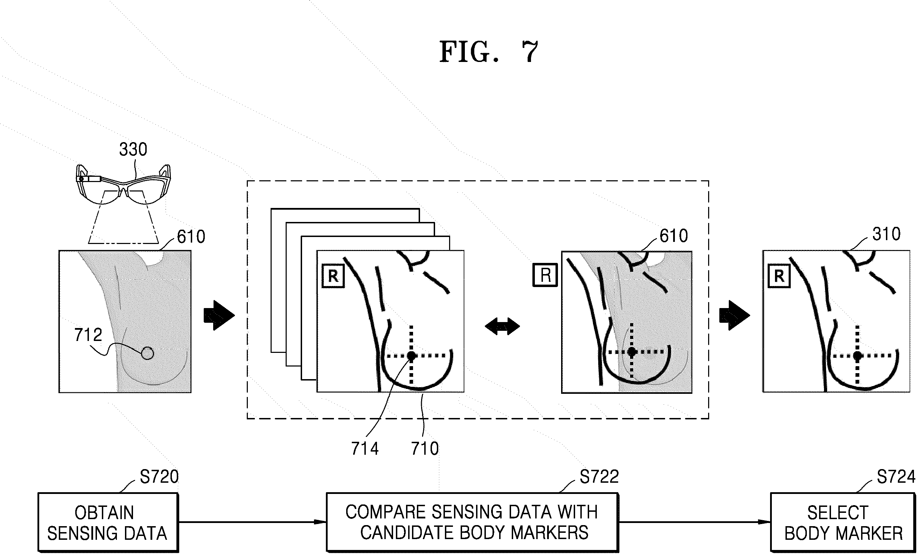

FIG. 7 shows a process of selecting a body marker from among a plurality of candidate body markers, according to an embodiment.

According to an embodiment, in operation S720, the ultrasound imaging apparatus 40 may obtain sensing data 610 generated by the sensing apparatus 330. According to an embodiment, the sensing data 610 may include an anatomical feature of a scanning part. The ultrasound imaging apparatus 40 may recognize the anatomical feature (for example, the nipples of the breast) 712 of the scanning part from the sensing data 610.

Then, in operation S722, the ultrasound imaging apparatus 40 may compare the sensing data 610 with a plurality of candidate body markers 710. The plurality of candidate body markers 710 may have been stored in advance in the memory 150. The plurality of candidate body markers 710 may include position information of anatomical features 714 of the scanning part. Also, the plurality of candidate body markers 710 may include attribute information of the respective candidate body markers 710. The attribute information may include attributes, such as, for example, left breast, right breast, excised left breast, excised right breast, Relaxed, Round, Side Set, Slender, Asymmetric, Athletic, Bell, East West, or Tear Drop. Also, the attribute information may include position information, such as supine position, right lateral position, left lateral position, and sitting position. Also, the attribute information may include childbirth/breastfeeding information, such as before giving birth, during pregnancy, during breastfeeding, and after giving birth.

The ultrasound imaging apparatus 40 may compare the sensing data 610 with the plurality of candidate body markers 710 to determine similarity between the sensing data 610 and the plurality of candidate body markers 710. The ultrasound imaging apparatus 40 may determine similarity between the sensing data 610 and the plurality of candidate body markers 710 by using a position of the anatomical feature 712 of the sensing data 710 and positions of the anatomical features 714 of the candidate body markers 710. Also, according to an embodiment, the ultrasound imaging apparatus 40 may select some candidate body markers 710 from among the plurality of candidate body markers 710 by using the attribute information included in the candidate body markers 710 and attribute information of the sensing data 610, and compare the sensing data 610 with the selected candidate body markers 710.

Then, the ultrasound imaging apparatus 40 may select a candidate body marker 710 having highest similarity to the sensing data 610 from among the selected candidate body markers 710, as a body marker 310, in operation S724.

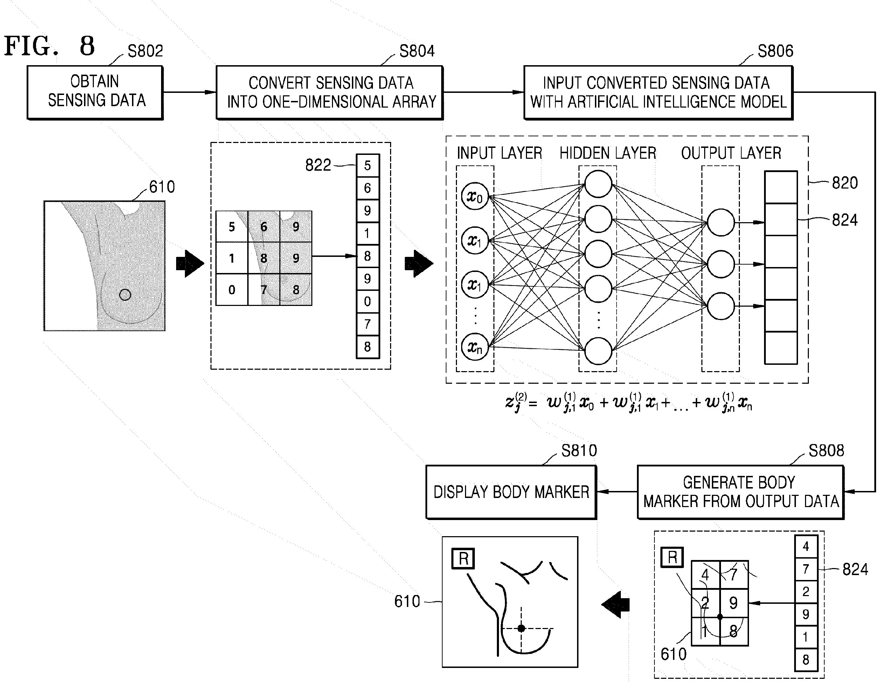

FIG. 8 shows a process of generating a body marker by using an artificial intelligence (Al) model, according to an embodiment.

According to an embodiment, the ultrasound imaging apparatus 40 may generate a body marker 310 from sensing data 610 by using an Al model. The ultrasound imaging apparatus 40 may use a first AI model 820 trained to generate the body marker 310 from the sensing data 610.

According to an embodiment, the ultrasound imaging apparatus 40 may store the first AI model 820 and execute the first AI model 820.

Also, according to an embodiment, a preset server may store the first AI model 820 and execute the first AI model 820. In this case, the ultrasound imaging apparatus 40 may transmit the sensing data 610 to the preset server and the preset server may input the sensing data 610 to the first AI model 820 to obtain the body marker 310. The preset server may transmit the obtained body marker 310 to the ultrasound imaging apparatus 40, and the ultrasound imaging apparatus 40 may use the body marker 310 received from the preset server.

Also, according to an embodiment, the sensing apparatus 330 may store the first AI model 820 and execute the first AI model 820. The sensing apparatus 330 may input the sensing data 610 to the first AI model 820 and obtain the body marker 310 from the first AI model 820.

The first AI model 820 may correspond to a machine-learned Deep Neural Network (DNN). The first AI model 820 may be a model machine-learned by using a plurality of pieces of sensing data 610 and a plurality of body markers 310 as learning data. According to an embodiment, the first AI model 820 may use a Multilayer Perceptron method (MLP). Also, according to an embodiment, the first AI model 820 may be implemented by using a model, such as Convolutional Neural Network (CNN), Recurrent Neural Network (RNN), or Generative Adversarial Network (GAN), Long Short-Term Memory (LSTM), etc.

Firs, in operation S802, the ultrasound imaging apparatus 40 may obtain sensing data 610. The ultrasound imaging apparatus 40 may obtain the sensing data 610 from the sensing apparatus 330.

Then, in operation S804, the ultrasound imaging apparatus 40 may convert the sensing data 610 into input data 8322 in the form of a one-dimensional array. The ultrasound imaging apparatus 40 may generate the input data 822 in the form of the one-dimensional array based on respective pixel values of the sensing data 610. According to an embodiment, the first AI model 820 may receive the input data 822 of n elements and output output data 824 of m elements. The ultrasound imaging apparatus 40 may generate the input data 822 by converting the sensing data 610 into the form of a one-dimensional array of n elements. The ultrasound imaging apparatus 40 may convert the pixel values of the sensing data 610 into the form of a one-dimensional array of n elements. For example, according to sensing data 610 having 256 pixels and input data 822 having 16 elements, the ultrasound imaging apparatus 40 may generate the input data 822 by converting a value of each block including 16 pixels into one representative value.

Then, in operation S806, the ultrasound imaging apparatus 40 may input the input data 822 to a first AI model 820. The first AI model 820 may transfer the input data 822 in the form of the one-dimensional array to an input layer of the M LP. The input data 822 in the form of the one-dimensional array input to the input layer may be propagated to a pre-trained hidden layer, multiplied by weights, computed by a linear combination, and converted into new intermediate values or result values. At this time, the first AI model 820 may include a plurality of hidden layers. The first AI model 820 may generate the output data 824 including the m elements.

Then, in operation S808, the ultrasound imaging apparatus 40 may generate a body marker 610 from the output data 824. The ultrasound imaging apparatus 40 may convert the output data 824 into a body marker 610. According to an embodiment, the output data 824 may correspond to a pixel value representing a visual shape of the body marker 610. In this case, the ultrasound imaging apparatus 40 may generate the body marker 610 by selecting a candidate body marker with a shape corresponding to the output data 824. Also, according to an embodiment, the output data 824 may correspond to an identifier for selecting one from among a plurality of candidate body markers. In this case, the ultrasound imaging apparatus 40 may select a candidate body marker selected by the output data 824 as the body marker 610.

Then, in operation S810, the ultrasound imaging apparatus 40 may display the body marker 610 generated from the output data 824.

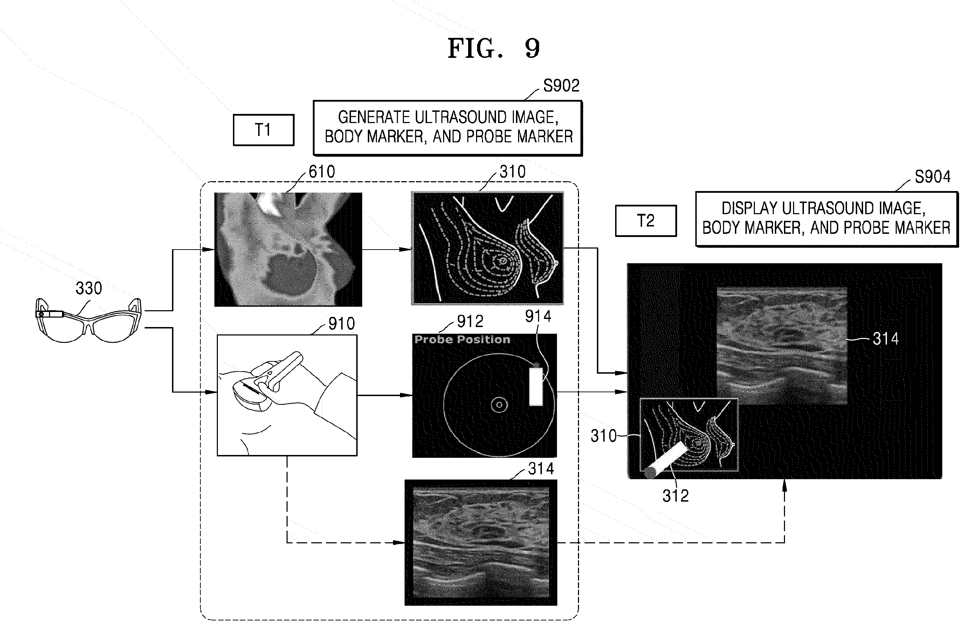

FIG. 9 shows a process of generating an ultrasound image, a body marker, and a probe marker, synchronized with each other, according to an embodiment.

According to an embodiment, the ultrasound imaging apparatus 40 may synchronize an ultrasound image 314, a body marker 310, and a probe marker 312 generated at a first time T1, with each other, and display the ultrasound image 314, the body marker 310, and the probe marker 312 at a second time T2.

The ultrasound imaging apparatus 40 may generate a body marker 310 of the first time T1 by using sensing data 610 generated by the sensing apparatus 330 in step S902. Also, the ultrasound imaging apparatus 40 may generate a probe position image 912 by using real-time sensing data 910 of the first time T1.

The sensing data 610 may be data obtained before a patient is scanned with the probe 20. The ultrasound imaging apparatus 40 may generate the body marker 310 by using the sensing data 610 captured from the patient who is ready to be scanned, while the probe 20 is not placed. The body marker 310 may be used until the patient's position changes.

The real-time sensing data 910 may be obtained by the sensing apparatus 330 after the probe 20 is placed on a scanning part of the patient. According to an embodiment, the sensing data 610 may be data obtained at the time T11 after the patient is ready to be scanned, and the real-time sensing data 910 may correspond to data obtained at the time T12 while scanning is performed by using the probe 20 after the first time T11. The ultrasound imaging apparatus 40 may use the body marker 310 obtained at the time T11 while the patient's position does not change.

The ultrasound imaging apparatus 40 may generate a probe position image 912 by using the real-time sensing data 910. The probe position image 912 may be an image representing a position of the probe 20 based on an anatomical feature of a scanning part. For example, the probe position image 912 may display a probe indicator 914 representing a position of the probe 20 based on positions of the nipples of the breast. The ultrasound imaging apparatus 40 may locate a probe marker 312 on the body marker 310 of the time T11 while the patient maintains the same position. The ultrasound imaging apparatus 40 may define a position corresponding to a position of the probe indicator 914 on the body marker 310 of the time T11, and locate the probe marker 312 at the defined position.

Also, the ultrasound imaging apparatus 40 may obtain an ultrasound image 314 from ultrasound data generated by the probe 20.

The ultrasound imaging apparatus 40 may synchronize the body marker 310, the probe position image 912, and the ultrasound image 314 with each other. The ultrasound imaging apparatus 40 may synchronize a time of the probe position image 912 and a time of the ultrasound image 314 with the time T12. Also, the ultrasound imaging apparatus 40 may use the body marker 310 of the time T11 while the patient maintains the same position.

Then, in operation S904, the ultrasound imaging apparatus 40 may display the ultrasound image 314, the body marker 310, and the probe marker 312, synchronized with the second time T2. According to an embodiment, a time delay may occur between the time T12 and the second time T2 due to a time delay caused by an image processing operation of the ultrasound imaging apparatus 40, a time delay caused by a communication operation between the sensing apparatus 330 and the ultrasound imaging apparatus 40, etc. Also, according to an embodiment, the ultrasound imaging apparatus 40 may store, after capturing the ultrasound image 314 at the time T12, the ultrasound image 314 together with the body marker 310 and the probe marker 312 and then, display the ultrasound image 314, the body marker 310, and the probe marker 312 at the second time T2.

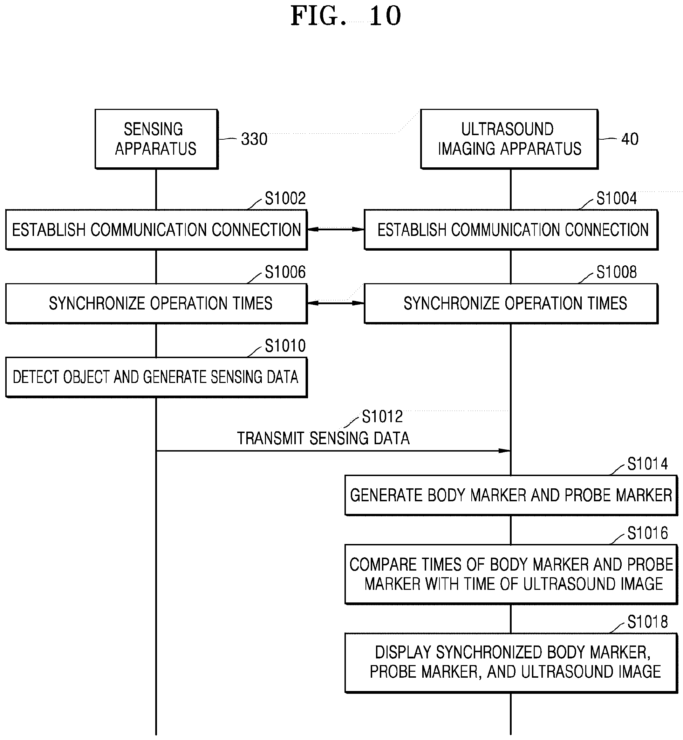

FIG. 10 is a flowchart illustrating a method of synchronizing a body marker, a probe marker, and an ultrasound image with each other, according to an embodiment.

According to an embodiment, by synchronizing an operation time of the ultrasound imaging apparatus 40 with an operation time of the sensing apparatus 330, a body marker, a probe marker, and an ultrasound image may be synchronized with each other.

First, in operations S1002 and S1004, the sensing apparatus 330 and the ultrasound imaging apparatus 40 may establish a communication connection. For example, the sensing apparatus 330 and the ultrasound imaging apparatus 40 may establish a communication connection through Bluetooth pairing.

Then, in operations S1006 and S1008, the sensing apparatus 330 and the ultrasound imaging apparatus 40 may synchronize operation times. For example, the sensing apparatus 330 may synchronize the operation time by receiving operation time information of the ultrasound imaging apparatus 40 and synchronizing operation time information of the sensing apparatus 330 with the operation time information of the ultrasound imaging apparatus 40. Also, the ultrasound imaging apparatus 40 may synchronize the operation time by receiving operation time information of the sensing apparatus 330 and synchronizing operation time information of the ultrasound imaging apparatus 40 with the operation time information of the sensing apparatus 330.

After the synchronization of the operation times is completed, the sensing apparatus 330 may detect an object and generate sensing data, in operation S1010. The sensing apparatus 330 may add information about a time at which the sensing data has been obtained, to the sensing data.

Then, in operation S1012, the sensing apparatus 330 may transfer the sensing data to the ultrasound imaging apparatus 40 through the established communication connection. The sensing data may include information about a time at which the sensing data has been obtained.

Then, in operation S1014, the ultrasound imaging apparatus 40 may generate a body marker and a probe marker from the sensing data.

Meanwhile, the ultrasound imaging apparatus 40 may generate an ultrasound image. The ultrasound image may include time information about a time at which the ultrasound image has been captured. The ultrasound imaging apparatus 40 may compare the time of the sensing data with the information about the captured time of the ultrasound image, in operation S1016. The time information of the sensing data may correspond to time information of the body marker and the probe marker.

Then, in operation S1018, the ultrasound imaging apparatus 40 may synchronize the body marker, the probe marker, and the ultrasound image with each other and display the body marker, the probe marker, and the ultrasound image. As described above, while a patient maintains the same position, a latest body marker may be used, and a probe marker and an ultrasound image may be time-synchronized and displayed.

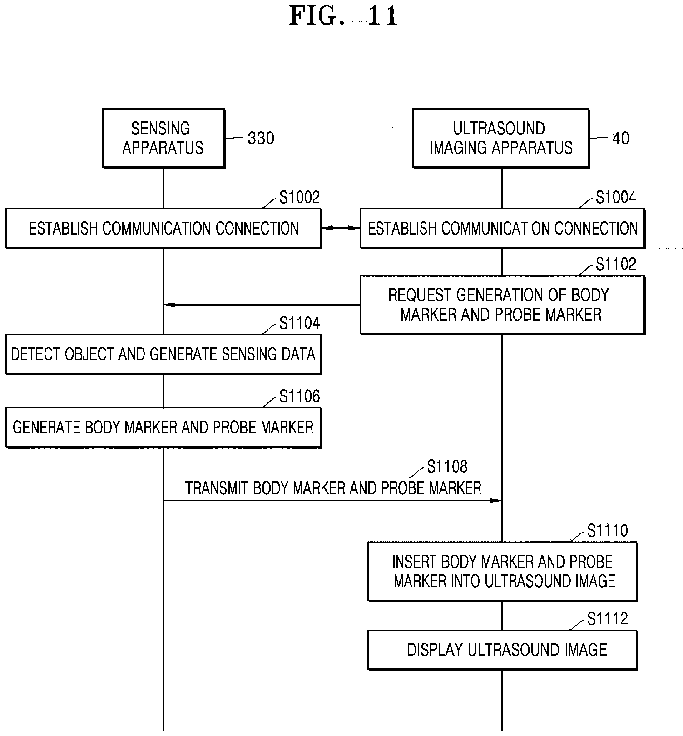

FIG. 11 is a flowchart illustrating a method of displaying a body marker, a probe marker, and an ultrasound image, according to an embodiment.

According to an embodiment, a body marker and a probe marker may be inserted into an ultrasound image and displayed.

First, in operations S1002 and S1004, the sensing apparatus 330 and the ultrasound imaging apparatus 40 may establish a communication connection. For example, the sensing apparatus 330 and the ultrasound imaging apparatus 40 may establish a communication connection through Bluetooth pairing.

Then, in operation S1102, the ultrasound imaging apparatus 40 may transmit a request for generating a body marker and a probe marker to the sensing apparatus 330. While the ultrasound imaging apparatus 40 captures an ultrasound image by using the probe 20, the ultrasound imaging apparatus 40 may request the sensing apparatus 330 for a body marker and a probe marker that are to be inserted into the ultrasound image.

In operation S1104, the sensing apparatus 330 may detect an object and generate sensing data. The sensing apparatus 330 may generate the sensing data in response to reception of the request for generating a body marker and a probe marker from the ultrasound imaging apparatus 40. The sensing data may include time information about a time at which the sensing data is obtained.

Then, in operation S1106, the sensing apparatus 330 may generate a body marker and a probe marker. The sensing apparatus 330 may insert the time information about the time at which the sensing data is obtained into the body marker and the probe marker.

Then, in operation 1108, the sensing apparatus 330 may transmit the body marker and the probe marker to the ultrasound imaging apparatus 40 through the established communication connection.

The ultrasound imaging apparatus 40 may insert the body marker and the probe marker received from the sensing apparatus 330 into the ultrasound image, in operation S1110. According to an embodiment, the ultrasound imaging apparatus 40 may insert the received body marker and probe marker into the ultrasound image at a time at which the ultrasound imaging apparatus 40 has transmitted the request for generating the body marker and the probe marker to the sensing apparatus 330. Also, according to an embodiment, the ultrasound imaging apparatus 40 may synchronize the ultrasound image with the body marker and the probe marker with each other, based on the time information inserted into the body marker and the probe marker, and insert the synchronized body marker and probe marker into the ultrasound image.

According to an embodiment, the ultrasound imaging apparatus 40 may insert the body marker and the probe marker as meta information of the ultrasound image into the file of the ultrasound image. Also, according to an embodiment, the ultrasound imaging apparatus 40 may store the body marker and the probe marker as separate files, and insert file information of the body marker and the probe marker as meta data of the ultrasound image into the file of the ultrasound image. Also, according to an embodiment, the ultrasound imaging apparatus 40 may synthesize the ultrasound image with the body marker and the probe marker and store the synthesized image as an image file.

Then, in operation S1112, the ultrasound imaging apparatus 40 may display the ultrasound image into which the body marker and the probe marker have been inserted. According to an embodiment, the ultrasound imaging apparatus 40 may display the body marker and the probe marker by using meta information of the ultrasound image file. Also, according to an embodiment, the ultrasound imaging apparatus 40 may display the ultrasound image into which the body marker and the probe marker have been synthesized.

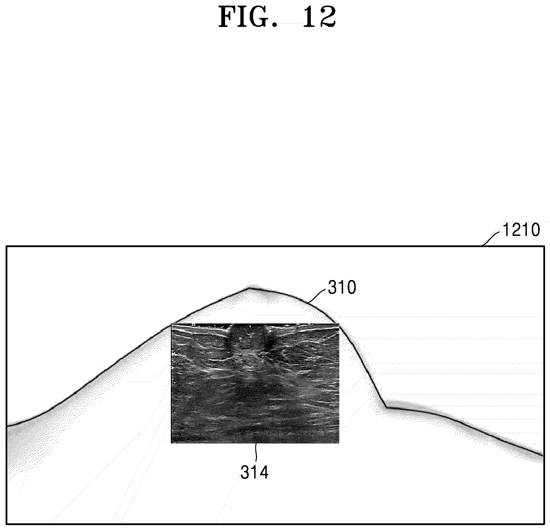

FIG. 12 shows a Graphic User Interface (GUI) for displaying a body marker and an ultrasound image, according to an embodiment.

According to an embodiment, the ultrasound imaging apparatus 40 may display a body marker 310 and an ultrasound image 314 on a GUI view 1210 in such a way as to overlap the ultrasound image 314 with the body marker 310. The ultrasound imaging apparatus 40 may generate the body marker 310 in a preset direction based on sensing data. The ultrasound imaging apparatus 40 may display the body marker 310 and the ultrasound image 314 in such a way as to overlap the ultrasound image 314 with the body marker 310 by reflecting a direction in which the probe 30 has captured the ultrasound image 314. For example, according to the ultrasound image 314 being captured by scanning a lateral side of the breast with the probe 20, the ultrasound imaging apparatus 40 may generate a body marker 310 representing the lateral side of the breast and display the ultrasound image 314 on a lateral view of the body marker 310.

According to the ultrasound image 314 being photographed by scanning a front side of the breast with the probe 20, the ultrasound imaging apparatus 40 may generate a body marker 310 representing a front side of the breast and display the ultrasound image 314 and the body marker 310 in such a way as to overlap the ultrasound image 314 on the body marker 310. The ultrasound imaging apparatus 40 may locate the ultrasound image 314 on the body marker 310 by reflecting a position of the probe 20 at which the ultrasound image 314 has been captured. For example, according to the ultrasound image 314 being captured at the right side from the nipples of the breast, the ultrasound image 314 may be displayed at the right side from the nipples of the breast in the body marker 310.

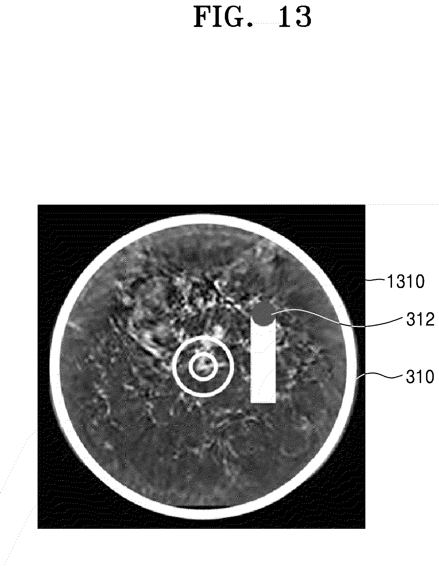

FIG. 13 shows a configuration of displaying a medical image of another modality, a body marker, and a probe marker, according to an embodiment.

According to an embodiment, the ultrasound imaging apparatus 40 may display a medical image of another modality, a body marker, and a probe marker, together. The ultrasound imaging apparatus 40 may display the medical image of the other modality, while displaying an ultrasound image. For example, the medical image 1310 may correspond to a CT image, a MRI image, or an X-ray image. In the case in which there is a medical image 1310 of another modality corresponding to an ultrasound image, the ultrasound imaging apparatus 40 may display the medical image 1310 and a body marker 310 and a probe marker 312 corresponding to the ultrasound image in such a way as to overlap the body marker 310 and the probe marker 312 with the medical image 1310. According to an embodiment, the ultrasound imaging apparatus 40 may identify a position corresponding to the body marker 310 in the medical image 1310 and locate the body marker 310 with the corresponding size at the corresponding position. Also, the ultrasound imaging apparatus 40 may identify a position and an orientation corresponding to the probe marker 312 in the medical image 1310, and locate the probe marker 312 at the corresponding position and orientation.

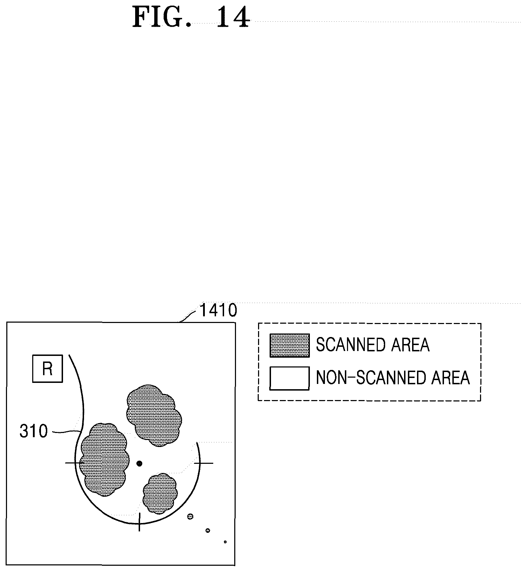

FIG. 14 shows a GUI for representing scan information on a body marker, according to an embodiment.

According to an embodiment, the ultrasound imaging apparatus 40 may provide scan information 1410 representing a scanned area scanned with the probe 20 and a non-scanned area not scanned with the probe 20, on the body marker 310. The scanned area may represent an area which has been scanned with the probe 20 and of which an ultrasound image has been obtained. The non-scanned area may represent an area of which an ultrasound image has not yet been obtained. According to an embodiment, the ultrasound imaging apparatus 40 may track a scanned area and a non-scanned area in a scanning part, accumulate the scanned area and the non-scanned area, and then, define the scanned area and the non-scanned area. Also, the ultrasound imaging apparatus 40 may display the scanned area and the non-scanned area on the body marker 310, as shown in FIG. 14. The scanned area and the non-scanned area may be expressed using attributes, such as color, shading, or pattern. Also, the ultrasound imaging apparatus 40 may represent and display a scanned area indicator on the scanned area.

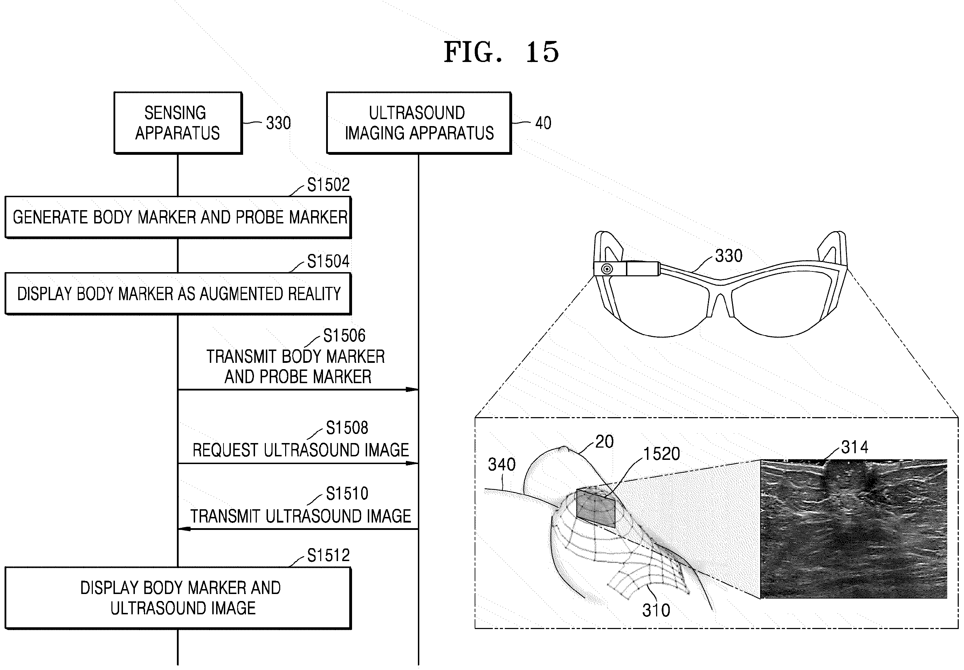

FIG. 15 is a flowchart illustrating a process of displaying a body marker and an ultrasound image in a sensing apparatus, according to an embodiment.

According to an embodiment, the sensing apparatus 330 may correspond to a glass apparatus. The sensing apparatus 330 may display a body marker 310 overlapping with a scanning part of a patient 340, as an Augmented Reality (AR) object, while photographing the patient 340. Also, according to an embodiment, the sensing apparatus 330 may display an ultrasound image 314 as an AR object.

First, in operation S1502, the sensing apparatus 330 may generate a body marker 310 and a probe marker from sensing data. The sensing apparatus 330 may generate a three-dimensional body marker 310 corresponding to a scanning part of a patient 340.

Then, in operation S1504, the sensing apparatus 330 may display the body marker 310 on the scanning part of the patient 340. The sensing apparatus 330 may overlap the body marker 310 with the scanning part to correspond the body marker 310 to the scanning part of the patient 340. The body marker 310 may be displayed as an AR object on the scanning part. The scanning part of the patient 340 may be an actual object that is seen through glass of the sensing apparatus 330, and the body marker 310 may be an AR object displayed overlapping with the scanning part of the patient 340. The sensing apparatus 330 may photograph the scanning part with a camera, recognize the scanning part, and display the AR object in such a way as to overlap the AR object with the scanning part.

Then, in operation S1506, the sensing apparatus 330 may transmit the body marker 310 and the probe marker to the ultrasound imaging apparatus 40. Also, in operation S1508, the sensing apparatus 330 may request the ultrasound imaging apparatus 40 for an ultrasound image 314.

In operation S1510, the ultrasound imaging apparatus 40 may transmit the ultrasound image 314 to the sensing apparatus 330 in response to the request for the ultrasound image 314.

In operation S1512, the sensing apparatus 330 may receive the ultrasound image 314 from the ultrasound imaging apparatus 40 and display the ultrasound image 314 as an AR object. The sensing apparatus 330 may display the body marker 310 and the ultrasound image 314 as AR objects, together. The sensing apparatus 330 may synchronize the body marker 310 with the ultrasound image 314 and then, display the body marker 310 and the ultrasound image 314. Also, the sensing apparatus 330 may be synchronized with the ultrasound imaging apparatus 40 and then, display the ultrasound image 314.

According to an embodiment, the sensing apparatus 330 may display a reduced image of the ultrasound image 314 in a scan area 1520 corresponding to a scan area of the probe 20. The scan area 1520 may be an area corresponding to a position of the probe marker. According to an embodiment, by displaying the reduced image of the ultrasound image 314 on the scan area 1520, a user may intuitively recognize the scan area 1520 of the probe 20.

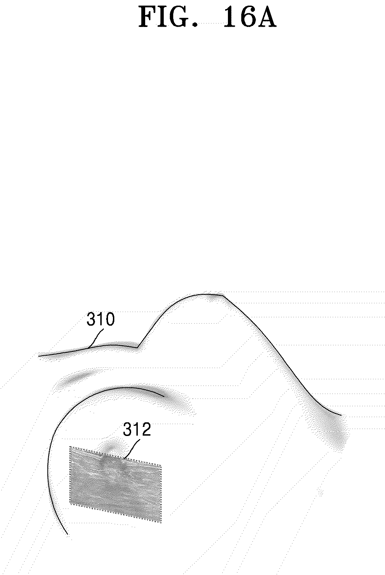

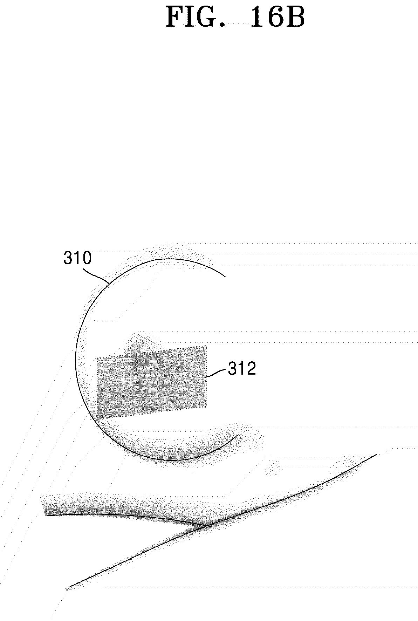

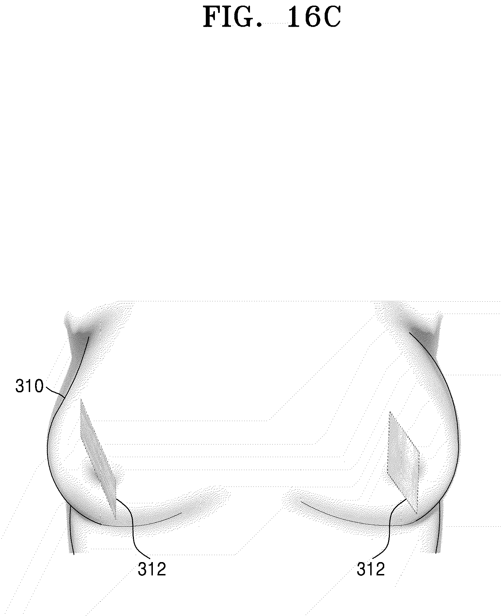

FIGS. 16A, 16B, and 16C show body markers and probe markers according to an embodiment.

According to an embodiment, the sensing apparatus 330 may generate a body marker 310 having a shape corresponding to a patient. The body marker 310 may be generated by reflecting the patient's position, a shape of a scanning part, and a scanning direction, as shown in FIGS. 16A, 16B, and 16C. Also, the body marker 310 may be generated in three dimensions, as shown in FIGS. 16A, 16B, and 16C.

Also, according to an embodiment, the sensing apparatus 330 may display the probe marker 312 in an area corresponding to a position of the probe 20 on the body marker 310. The probe marker 312 may reduce a synchronized ultrasound image and display the reduced ultrasound image.

According to an embodiment, the sensing apparatus 330 may display probe markers 312 of two times, as shown in FIG. 16C. Each probe marker 312 may include a reduced image of an ultrasound image corresponding to a scanning time of the probe marker 312.

According to an embodiment, the sensing apparatus 330 may correspond to a glass apparatus. The glass apparatus may display the probe marker 312 as an AR object on an object that is actually seen through the glass device. The glass device may display an AR object corresponding to the probe marker 312 at a position corresponding to a position of a probe on the object. The glass device may display the probe marker 312 without displaying the body marker 310.

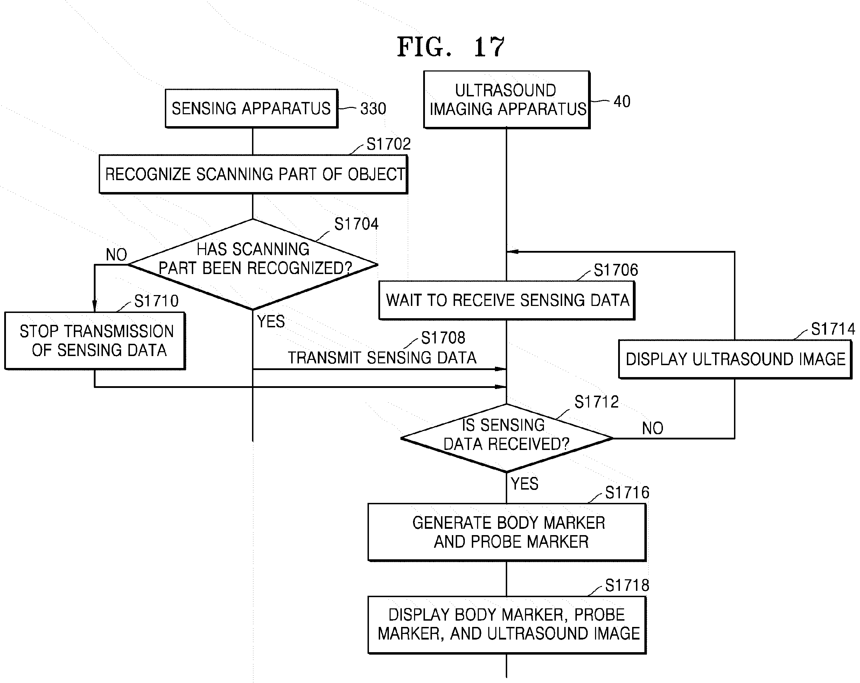

FIG. 17 shows a process of obtaining sensing data and obtaining a body marker and a probe marker, according to an embodiment.

According to an embodiment, while a user does not look at a scanning part of an object, the sensing apparatus 330 may stop transmitting sensing data to the ultrasound imaging apparatus 40 and the ultrasound imaging apparatus 40 may stop displaying a body marker and a probe marker.

First, in operation S1702, the sensing apparatus 330 may recognize a scanning part of an object. The sensing apparatus 330 may recognize the scanning part of the object by using sensing data. The sensing apparatus 330 may recognize the scanning part of the object by using a preset object recognition algorithm. Also, according to an embodiment, the sensing apparatus 330 may recognize the scanning part of the object by using an AI model.

Then, in operation S1704, the sensing apparatus 330 may determine whether the scanning part of the object has been recognized from the sensing data.

According to the scanning part of the object having been recognized from the sensing data, the sensing apparatus 330 may transmit the sensing data to the ultrasound imaging apparatus 40, in operation S1708. While the ultrasound imaging apparatus 40 waits to receive sensing data in operation S1706, the ultrasound imaging apparatus 40 may receive the sensing data from the sensing apparatus 330.

According to the scanning part of the object having not been recognized from the sensing data, the sensing apparatus 330 may stop transmission of sensing data in operation S1710. In an embodiment, according to the sensing apparatus 330 stopping transmission of sensing data, the sensing apparatus 330 may transmit information indicating that transmission of sensing data has stopped, to the ultrasound imaging apparatus 40.

The ultrasound imaging apparatus 40 may determine whether sensing data has been received from the sensing apparatus 330, in operation S1712.

In the case in which no sensing data has been received, the ultrasound imaging apparatus 40 may display an ultrasound image without a body marker and a probe marker, in operation S1714.

In the case in which sensing data has been received, the ultrasound imaging apparatus 40 may generate a body marker and a probe marker, in operation S1716. Then, the ultrasound imaging apparatus 40 may display the body marker, the probe marker, and the ultrasound image, in operation S1718.

A computer-readable storage medium may be provided in the form of a non-transitory storage medium. Herein, the term 'non-transitory storage medium' simply means that the storage medium is a tangible device, and does not include a signal (e.g., an electromagnetic wave), but this term does not differentiate between where data is semi-permanently stored in the storage medium and where the data is temporarily stored in the storage medium. For example, the 'non-transitory storage medium' may include a buffer in which data is temporarily stored.

According to an embodiment, the method according to various embodiments disclosed in this specification may be included in a computer program product and provided. The computer program product may be traded between a seller and a purchaser as a commodity. The computer program product may be distributed in the form of a machine-readable storage medium (e.g., compact disc read only memory (CD-ROM)), or be distributed (e.g., downloadable or uploadable) online via an application store (e.g., Play Store^{™}) or between two user devices (e.g., smart phones) directly. When distributed online, at least part of the computer program product may be temporarily generated or at least temporarily stored in the machine-readable storage medium, such as a memory of the manufacturer's server, a server of the application store, or a relay server.

## Claims

1. An ultrasound imaging apparatus comprising:
a probe configured to transmit an ultrasound signal to an object and detect an echo signal reflected from the object;
a display;
a communication module;
a memory storing at least one instruction; and
at least one processor configured to execute the at least one instruction to
generate a first ultrasound image from ultrasound data generated by the probe,
receive sensing data generated by detecting at least one part of the object and the probe from a sensing apparatus through the communication module,
generate, based on the sensing data, a body marker and a probe marker, the body marker representing a scanning part of the object and the probe marker being positioned on the body marker to represent a position of the probe on the scanning part of the object,
display the first ultrasound image, the body marker, and the probe marker on the display, and
change the body marker based on at least one of a shape, a type, or a scanning direction of the scanning part or a patient's position.

2. The ultrasound imaging apparatus of claim 1, wherein
the at least one processor is further configured to execute the at least one instruction to
update the body marker and the probe marker based on the sensing data,
synchronize the body marker, the probe marker, and the ultrasound image with each other, and display the body marker, the probe marker, and the ultrasound image.

3. The ultrasound imaging apparatus of any one of claims 1 to 2, wherein
the at least one processor is further configured to execute the at least one instruction to select, based on the sensing data, a candidate body marker from among a plurality of candidate body markers as a body marker, and locate the probe marker on the selected body marker,
wherein the plurality of candidate body markers are expressed differently according to at least one among a shape, a type, or a scanning direction of the scanning part or a patient's position.

4. The ultrasound imaging apparatus of any one of claims 1 to 3, wherein
the body marker and the probe marker are generated by the sensing apparatus, and
the at least one processor is further configured to execute the at least one instruction to
receive the body marker and the probe marker generated by the sensing apparatus through the communication module, and
display the received body marker and the received probe marker on the display.

5. The ultrasound imaging apparatus of any one of claims 1 to 4, wherein
the at least one processor is further configured to execute the at least one instruction to
synchronize an operation time of the sensing apparatus with an operation time of the ultrasound imaging apparatus,
synchronize an acquisition time of the sensing data with an acquisition time of the first ultrasound image, and
display the body marker and the probe marker generated from the synchronized sensing data together with the first ultrasound image.

6. The ultrasound imaging apparatus of any one of claims 1 to 5, wherein
the at least one processor is further configured to execute the at least one instruction to synchronize the body marker, the probe marker, and the first ultrasound image with each other by using time information included in the sensing data and time information included in the first ultrasound image.

7. The ultrasound imaging apparatus of any one of claims 1 to 6, wherein
the sensing apparatus comprises a glass device including a detecting sensor, and
the detecting sensor includes at least one of an image sensor, an infrared sensor, a thermal imaging camera, an optical camera, a stereo camera, or an ultrasonic sensor.

8. The ultrasound imaging apparatus of claim 7, wherein
the glass device is configured to transmit the sensing data to the ultrasound imaging apparatus while a user's gaze is directed toward the scanning part, and stop transmitting the sensing data to the ultrasound imaging apparatus while the user's gaze is directed toward another part except for the scanning part, and
the at least one processor is further configured to execute the at least one instruction to
update the body marker and the probe marker based on the sensing data while receiving the sensing data, and
stop updating the body marker and the probe marker while reception of the sensing data stops.

9. The ultrasound imaging apparatus of any one of claims 1 to 8, wherein
the at least one processor is further configured to execute the at least one instruction to input the sensing data to a first artificial intelligence model to obtain the body marker,
wherein the sensing data is input to the first artificial intelligence model in a form of a one-dimensional array.

10. The ultrasound imaging apparatus of any one of claims 1 to 9, wherein
a position and an orientation of the probe marker on the body marker are defined based on an anatomical feature of the body marker.

11. The ultrasound imaging apparatus of any one of claims 1 to 10, wherein
the at least one processor is further configured to execute the at least one instruction to stop displaying the probe marker in response to determining, based on the sensing data, that the probe deviates from the scanning part.

12. The ultrasound imaging apparatus of any one of claims 1 to 11, wherein
the at least one processor is further configured to execute the at least one instruction to generate a Graphic User Interface (GUI) view that displays the body marker and the ultrasound image in such a way as to overlap the body marker with the ultrasound image, and display the GUI view on the display.

13. The ultrasound imaging apparatus of any one of claims 1 to 12, wherein
the at least one processor is further configured to execute the at least one instruction to display, on the body marker, a scan area indicator indicating an area scanned by the probe.

14. A method of controlling an ultrasound imaging apparatus, comprising:
generating a first ultrasound image from ultrasound data generated by a probe;
receiving, from a sensing apparatus, sensing data generated by detecting at least a part of an object and the probe;
generating, based on the sensing data, a body marker and a probe marker, the body marker representing a scanning part of the object and the probe marker being positioned on the body marker to represent a position of the probe on the scanning part of the object,
displaying the first ultrasound image, the body marker, and the probe marker, and
changing the body marker based on at least one of a shape, a type, or a scanning direction of the scanning part or a patient's position.

15. A computer-readable recording medium storing a program for causing a computer to perform the method of claim 14.

## Amended claims

### Amended claims in accordance with Rule 137(2) EPC.

1. An ultrasound imaging apparatus (40, 100) comprising:
a probe (20) configured to transmit an ultrasound signal to an object and detect an echo signal reflected from the object;
a display (140, 121, 122, 41);
a communication module (160);
a memory (150) storing at least one instruction; and
at least one processor (120) configured to execute the at least one instruction to generate a first ultrasound image from ultrasound data generated by the probe (20),
receive sensing data generated by detecting at least one part of the object and the probe from a sensing apparatus (330) through the communication module (160),
**characterized in that** the at least one processor (120) is further configured to:
generate, based on the sensing data, a body marker (310, 310a, 310b) and a probe marker (312), the body marker (310, 310a, 310b) representing a scanning part of the object and the probe marker (312) being positioned on the body marker (310, 310a, 310b) to represent a position of the probe (20) on the scanning part of the object, wherein the body marker (310, 310a, 310b) is drawings or image obtained by simplifying a shape of a scanning part of a patient based on the sensing data,
display the first ultrasound image, the body marker (310, 310a, 310b), and the probe marker (312) on the display (140, 121, 122, 41), and
change the body marker (310, 310a, 310b) based on a patient's position, wherein the patient's position comprises at least one of lying facing the ceiling, lying on the right side, or lying on the left side.

2. The ultrasound imaging apparatus (40, 100) of claim 1, wherein
the at least one processor (120) is further configured to execute the at least one instruction to
change the body marker (310, 310a, 310b) based on the patient's position, and at least one of: a shape, a type, or a scanning direction of the scanning part,
update the body marker (310, 310a, 310b) and the probe marker (312) based on the sensing data,
synchronize the body marker (310, 310a, 310b), the probe marker (312), and the ultrasound image with each other, and display the body marker (310, 310a, 310b), the probe marker (312), and the ultrasound image.

3. The ultrasound imaging apparatus (40, 100) of any one of claims 1 to 2, wherein
the at least one processor (120) is further configured to execute the at least one instruction to select, based on the sensing data, a candidate body marker from among a plurality of candidate body markers as a body marker, and locate the probe marker (312) on the selected body marker,
wherein the plurality of candidate body markers are expressed differently according to at least one among a shape, a type, or a scanning direction of the scanning part or a patient's position.

4. The ultrasound imaging apparatus (40, 100) of any one of claims 1 to 3, wherein
the body marker (310, 310a, 310b) and the probe marker (312) are generated by the sensing apparatus (330), and
the at least one processor (120) is further configured to execute the at least one instruction to
receive the body marker (310, 310a, 310b) and the probe marker (312) generated by the sensing apparatus (330) through the communication module (160), and
display the received body marker (310, 310a, 310b) and the received probe marker (312) on the display (140, 121, 122, 41).

5. The ultrasound imaging apparatus (40, 100) of any one of claims 1 to 4, wherein
the at least one processor (120) is further configured to execute the at least one instruction to
synchronize an operation time of the sensing apparatus (330) with an operation time of the ultrasound imaging apparatus (40, 100),
synchronize an acquisition time of the sensing data with an acquisition time of the first ultrasound image, and
display the body marker (310, 310a, 310b) and the probe marker (312) generated from the synchronized sensing data together with the first ultrasound image.

6. The ultrasound imaging apparatus (40, 100) of any one of claims 1 to 5, wherein
the at least one processor (120) is further configured to execute the at least one instruction to synchronize the body marker (310, 310a, 310b), the probe marker (312), and the first ultrasound image with each other by using time information included in the sensing data and time information included in the first ultrasound image.

7. The ultrasound imaging apparatus (40, 100) of any one of claims 1 to 6, wherein
the sensing apparatus (330) comprises a glass device including a detecting sensor, and
the detecting sensor includes at least one of an image sensor, an infrared sensor, a thermal imaging camera, an optical camera, a stereo camera, or an ultrasonic sensor.

8. The ultrasound imaging apparatus (40, 100) of claim 7, wherein
the glass device is configured to transmit the sensing data to the ultrasound imaging apparatus (40, 100) while a user's gaze is directed toward the scanning part, and stop transmitting the sensing data to the ultrasound imaging apparatus (40, 100) while the user's gaze is directed toward another part except for the scanning part, and
the at least one processor (120) is further configured to execute the at least one instruction to
update the body marker (310, 310a, 310b) and the probe marker (312) based on the sensing data while receiving the sensing data, and
stop updating the body marker (310, 310a, 310b) and the probe marker (312) while reception of the sensing data stops.

9. The ultrasound imaging apparatus (40, 100) of any one of claims 1 to 8, wherein
the at least one processor (120) is further configured to execute the at least one instruction to input the sensing data to a first artificial intelligence model to obtain the body marker (310, 310a, 310b),
wherein the sensing data is input to the first artificial intelligence model in a form of a one-dimensional array.

10. The ultrasound imaging apparatus (40, 100) of any one of claims 1 to 9, wherein
a position and an orientation of the probe marker (312) on the body marker (310, 310a, 310b) are defined based on an anatomical feature of the body marker (310, 310a, 310b).

11. The ultrasound imaging apparatus (40, 100) of any one of claims 1 to 10, wherein
the at least one processor (120) is further configured to execute the at least one instruction to stop displaying the probe marker (312) in response to determining, based on the sensing data, that the probe (20) deviates from the scanning part.

12. The ultrasound imaging apparatus (40, 100) of any one of claims 1 to 11, wherein
the at least one processor (120) is further configured to execute the at least one instruction to generate a Graphic User Interface (GUI) view that displays the body marker (310, 310a, 310b) and the ultrasound image in such a way as to overlap the body marker (310, 310a, 310b) with the ultrasound image, and display the GUI view on the display (140, 121, 122, 41).

13. The ultrasound imaging apparatus (40, 100) of any one of claims 1 to 12, wherein
the at least one processor (120) is further configured to execute the at least one instruction to display, on the body marker (310, 310a, 310b), a scan area indicator indicating an area scanned by the probe (20).

14. A method of controlling an ultrasound imaging apparatus (40, 100), comprising:
generating a first ultrasound image from ultrasound data generated by a probe (20);
receiving, from a sensing apparatus (330), sensing data generated by detecting at least a part of an object and the probe (20);
**characterized in that** the method further comprises:
generating, based on the sensing data, a body marker (310, 310a, 310b) and a probe marker (312), the body marker (310, 310a, 310b) representing a scanning part of the object and the probe marker (312) being positioned on the body marker (310, 310a, 310b) to represent a position of the probe (20) on the scanning part of the object, wherein the body marker (310, 310a, 310b) is drawings or image obtained by simplifying a shape of a scanning part of a patient based on the sensing data,
displaying the first ultrasound image, the body marker (310, 310a, 310b), and the probe marker (312), and
changing the body marker (310, 310a, 310b) based on a patient's position, wherein the patient's position comprises at least one of lying facing the ceiling, lying on the right side, or lying on the left side.

15. A computer-readable recording medium storing a program for causing a computer to perform the method of claim 14.
